# EUROPÄISCHE PATENTSCHRIFT

(11) **EP 2 734 176 B1**
(45) Veröffentlichungstag und Bekanntmachung des Hinweises auf die Patenterteilung: **14.12.2016**
(21) Anmeldenummer: 12725732.7
(22) Anmeldetag: 01.06.2012
(51) Int. Cl.: A61K 8/23, A61K 8/27, A61K 8/368, A61K 8/49, A61K 8/88, A61Q 5/00, A61Q 5/02

(54) **LEISTUNGSGESTEIGERTE WIRKSTOFFKOMBINATION UND HAARBEHANDLUNGSMITTEL GEGEN SCHUPPEN II**
HIGH-PERFORMANCE ACTIVE AGENT COMBINATION AND HAIR TREATMENT AGENT AGAINST DANDRUFF II
ASSOCIATION DE PRINCIPES ACTIFS À EFFET ACCRU ET AGENT DE TRAITEMENT CAPILLAIRE ANTIPELLICULAIRE II

(30) Priorität: 21.07.2011 DE 102011079539
(43) Veröffentlichungstag der Anmeldung: 28.05.2014
(73) Patentinhaber: Henkel AG & Co. KGaA, 40589 Düsseldorf (DE)
(72) Erfinder: SCHULZE ZUR WIESCHE, Erik, 20144 Hamburg (DE); POPPE, Elisabeth, 22763Hamburg (DE); SCHEUNEMANN, Volker, 21339 Lüneburg (DE)
(86) Internationale Anmeldenummer: PCT/EP2012/060403
(87) Internationale Veröffentlichungsnummer: WO 2013/010706

(56) Entgegenhaltungen:
- WO-A1-2012/055585
- WO-A1-2012/055586
- WO-A1-2012/055587
- WO-A1-2012/055588
- DE-A1- 10 111 288
- DE-A1-102006 037 113
- JP-A- 61 243 010
- JP-A- 62 221 616
- US-A- 5 302 323
- "Aq. suspension for treating dandruff-contains antibacterial (2)-zinc-pyridinechiol-(1)oxide and zinc oxide as stabiliser", WPI / THOMSON,, 4. August 1977 (1977-08-04), XP002670955,

## Beschreibung

Die Erfindung betrifft ein kosmetisches Haarbehandlungsmittel auf der Basis einer speziellen Wirkstoffkombinationen.

Reinigungsmittel für Haut und Haar, wie sie beispielsweise als flüssige Seifen, Shampoos, Duschbäder, Schaumbäder, Dusch- und Waschgele im Handel erhältlich sind, müssen nicht nur ein gutes Reinigungsvermögen aufweisen, sondern sollen weiterhin für die Haut und das Haar gut verträglich sein und auch bei häufiger Anwendung nicht zu starker Entfettung oder Trockenheit führen.

Einen wichtigen Aspekt bei der Haarreinigung und -pflege stellt die Bekämpfung von Schuppen dar, denn schon das leichte Auftreten von Schuppen auf der Kopfhaut oder anderen behaarten Körperregionen wird als Zeichen mangelnder Pflege angesehen. Darüber hinaus geht mit der Schuppenbildung meist ein Juckreiz einher, der als störend empfunden wird. Ein Juckreiz hingegen ruft Kratzreaktionen hervor, so dass es zu Verletzungen der betroffenen Hautpartien kommen kann, die wiederum die Basis für Infektionen und pathogene Erreger bilden können. Die Anforderungen an ein kosmetisches Mittel zur Schuppenbekämpfung sind daher hoch, denn zum einen soll die Kopfhaut gründlich und nachhaltig von Schuppen befreit werden, und zum anderen soll das Mittel für Haar und Kopfhaut gut verträglich sein.

Eine weitere, wichtige Anforderung ist eine möglichst schnelle Wirkung, so dass die oben genannte Kette von Nachteilen gar nicht erst auftreten.

Aus der Literatur sind eine Vielzahl von haarkosmetischen Mitteln bekannt, die die Schuppenbildung verhindern oder reduzieren sollen. Zur Auswahl stehen üblicherweise Mittel in der Form von Shampoos, Lotionen oder Haarwässern.

Aus der EP-A-348 015 ist die Verwendung von Zinksalzen als Antischuppenmittel bekannt.

Die Kombination dreier herkömmlicher und wirksamer Antischuppenmittel ist Gegenstand der EP 1 238 645 B1. Darin werden 1-(4-Chlorophenoxy)-1-(1 H-imidazolyl)-3,3-dimethyl-2-butanon (INCI-Bezeichnung: Climbazole), Bis(1-Hydroxy-2-(1H)-Pyridinethionato)Zinc (INCI-Bezeichnung: Zinc Pyrithione) und 1-Hydroxy-4-methyl-6-(2,4,4-trimethylpentyl)-2-(1H)pyridinone, 2-Aminoethanol Salz (INCI-Bezeichnung: Piroctone Olamine) bereits in niedrigen Einsatzkonzentrationen miteinander kombiniert, um ein Antischuppenmittel herzustellen, das die Bildung von Schuppen verhütet oder vermindert.

Die Dokumente JP-A-62 221 616 und JP-A-61 243 010 offenbaren Antischuppenmittel, welche Polylysin enthalten. Die DE-A-101 11 288 offenbart eine synergistische Wirkstoffkombination von Climbazol, Zinkpyrithion und Pirocton-Olamin. Thomson/WPI , 4.Aug.1977 ("Aq. suspension for treating dandruff contains antibacterial (2)-zinc-pyridinechiol-(1)-oxide and zinc oxide as stabiliser" XP002670955) schlägt Zinkoxid als Stabilisator für Zinkpyrithion vor.

Nachteilig an den Mitteln des Standes der Technik war bislang, dass sie zwar wirksam gegen Schuppen waren, diese Wirkung aber erst nach häufiger Anwendung eintrat.

Deshalb war es zusätzlich Aufgabe der vorliegenden Erfindung, ein schnell gegen Schuppen wirksames Mittel zu finden, das schonend zur Kopfhaut und dem Haar ist und das Haar im Hinblick auf seine Kämmbarkeit, seinen Glanz und seine Geschmeidigkeit nicht negativ beeinflusst. Darüber hinaus sollte die Antischuppenwirkung bekannter Wirkstoffe nach Möglichkeit nach Möglichkeit nicht nur beschleunigt, sondern auch gesteigert werden.

Diese Aufgabe wurde gelöst, indem mindestens zwei verschiedene Antischuppenwirkstoffe im Kombination mit einer speziellen Epsilon-Poly-L-Aminosäure in einem für die Anwendung auf behaarten Körperoberflächen geeigneten Mittel eingesetzt wurde.

Überraschenderweise konnte festgestellt werden, dass durch die Haarbehandlung mit kosmetischen Mitteln auf der Basis dieser Kombination eine deutlich schnellere und intensivere Wirkung eintrat als bei herkömmlichen Mitteln.

Gegenstand der Erfindung sind daher Haarbehandlungsmittel, enthaltend - bezogen auf ihr Gewicht -
a) 0,0001 bis 5 Gew.-% Epsilon-Poly-L-Lysin der allgemeinen Formel (I) 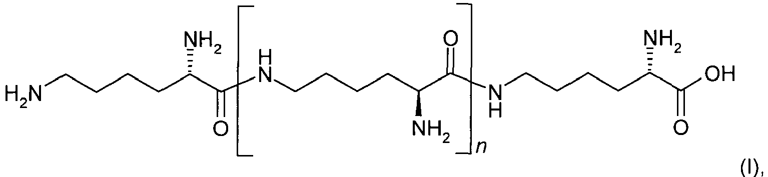 in der n für ganze Zahlen zwischen 5 und 100 steht,
b) 0,01 bis 10 Gew.-% eines Antischuppenwirkstoffes ausgewählt aus
   b1) Climbazol
   b2) Zinkpyrithion und/oder Zinkoxid
   b3) Pirocton-Olamin
   b4) Selensulfid(en)
   b5) Salicylsäure
c) 0,01 bis 10 Gew.-% eines Antischuppenwirkstoffes, der von b) verschieden ist und ausgewählt ist aus
   c1) Climbazol
   c2) Zinkpyrithion und/oder Zinkoxid
   c3) Pirocton-Olamin
   c4) Selensulfid(en)
   c5) Salicylsäure.
wobei das Mittel
- Epsilon-Poly-L-Lysin und Climbazol und Zinkpyrithion und/oder Zinkoxid
- Epsilon-Poly-L-Lysin und Climbazol und Pirocton-Olamin
- Epsilon-Poly-L-Lysin und Climbazol und Selensulfid(e)
enthält.

Als ersten wesentlichen Inhaltsstoff enthalten die erfindungsgemäßen Mittel Poly-L-Lysin, ein Oligo- bzw. Polymeres der natürlichen Aminosäure Lysin, das aufgrund seiner Verknüpfung in Epsilon-Position auch als Epsilon-Polylysin bezeichnet wird. Diese Verbindung führt zu einer Wirkungssteigerung und -beschleunigung des Antischuppenwirkstoffes, die über die eigene Antischuppenwirkung des Epsilon-Poly-L-lysins hinausgeht.

In bevorzugten erfindungsgemäßen Mitteln wird Epsilon-Poly-L-Lysin innerhalb engerer Mengenbereiche eingesetzt. Erfindungsgemäß bevorzugte Haarbehandlungsmittel sind dadurch gekennzeichnet, daß sie 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) enthalten.

Der Polymersiationsgrad der erfindungsgemäß eingesetzten Epsilon-Poly-L-Lysine liegt zwischen 5 und 100. Es konnte - je nach Haarbehandlungsmittel und Einsatzkonzentration - ein Wirkungsoptimum für Polymere innerhalb engerer Polymersiationsgrade beobachtet werden. Dementsprechend sind erfindungsgemäße Haarbehandlungsmittel bevorzugt, die Epsilon-Poly-L-Lysin der allgemeinen Formel (I) enthalten, in der n für ganze Zahlen von 10 bis 70, vorzugsweise von 15 bis 60, weiter bevorzugt von 20 bis 50 und insbesondere für die Zahlen 23, 24, 25, 26, 27, 28, 29, 30, 31, 32, 33, 34, 35 steht.

Ganz besonders bevorzugte erfindungsgemäße Haarbehandlungsmittel enthalten
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 23 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 24 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 25 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 26 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 27 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 28 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 29 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 30 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 31 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 32 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 33 und/oder
- 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) mit n = 34,
wobei die Gesamtmenge an Epsilon-Poly-L-Lysinen der allgemeinen Formel (I) erfindungsgemäß 0,0001 bis 5 Gew.-% beträgt.

Als zweiten wesentlichen Inhaltsstoff enthalten die erfindungsgemäßen Mittel 0,01 bis 10 Gew.-% eines Antischuppenwirkstoffes ausgewählt aus b1) Climbazol, b2) Zinkpyrithion und/oder Zinkoxid, b3) Pirocton-Olamin, b4) Selensulfid(en), b5) Salicylsäure. Diese Stoffe werden weiter unten ausführlich beschrieben.

Als dritten wesentlichen Inhaltsstoff enthalten die erfindungsgemäßen Mittel 0,01 bis 10 Gew.-% eines Antischuppenwirkstoffes, der von b) verschieden ist und ausgewählt aus b1) Climbazol, b2) Zinkpyrithion und/oder Zinkoxid, b3) Pirocton-Olamin, b4) Selensulfid(en), b5) Salicylsäure. Diese Stoffe werden weiter unten ausführlich beschrieben.

Bei der Auswahl der Inhaltstoffe b) und c) ist darauf zu achten, daß entweder b) oder c) Climbazol ist.

- Erfindungsgemäße Mittel enthalten
- Epsilon-Poly-L-Lysin und Climbazol und Zinkpyrithion und/oder Zinkoxid
- Epsilon-Poly-L-Lysin und Climbazol und Pirocton-Olamin
- Epsilon-Poly-L-Lysin und Climbazol und Selensulfid(e)

Es ist erfindungsgemäß ebenfalls möglich, zusätzlich zu den zwei zwingend enthaltenen voneinander verschiedenen Antischuppenwirkstoffen weitere optionale Antischuppenwirkstoffe einzusetzen. So sind erfindungsgemäße Mittel herstellbar, die drei oder vier oder fünf voneinander verschiedene Antischuppenwirkstoffe enthalten.

Besonders bevorzugte erfindungsgemäße Mittel enthalten
- Epsilon-Poly-L-Lysin und Climbazol und Zinkpyrithion und/oder Zinkoxid und Pirocton-Olamin
- Epsilon-Poly-L-Lysin und Climbazol und Zinkpyrithion und/oder Zinkoxid und Selensulfid(e)
- Epsilon-Poly-L-Lysin und Climbazol und Zinkpyrithion und/oder Zinkoxid und Salicylsäure
- Epsilon-Poly-L-Lysin und Climbazol und Pirocton-Olamin und Selensulfid(e)
- Epsilon-Poly-L-Lysin und Climbazol und Pirocton-Olamin und Salicylsäure
- Epsilon-Poly-L-Lysin und Climbazol und Selensulfid(e) und Salicylsäure

Weiter bevorzugte erfindungsgemäße Mittel enthalten
- Epsilon-Poly-L-Lysin und Climbazol und Zinkpyrithion und/oder Zinkoxid und Pirocton-Olamin und Selensulfid(e)
- Epsilon-Poly-L-Lysin und Climbazol und Zinkpyrithion und/oder Zinkoxid und Pirocton-Olamin und Salicylsäure
- Epsilon-Poly-L-Lysin und Climbazol und Pirocton-Olamin und Selensulfid(e) und Salicylsäure
- Epsilon-Poly-L-Lysin und Climbazol und Zinkpyrithion und/oder Zinkoxid und Selensulfid(e) und Salicylsäure

Ganz besonders bevorzugte erfindungsgemäße Mittel enthalten Epsilon-Poly-L-Lysin und Climbazol und Zinkpyrithion und/oder Zinkoxid und Pirocton-Olamin und Selensulfid(e) und Salicylsäure.

Die erfindungsgemäßen Mittel enthalten 0,01 bis 10 Gew.-% Climbazol. Climbazol ist eine chemische Verbindung, die antimykotisch und fungistatisch wirkt. Es kommt in Form eines Racemats in den Handel

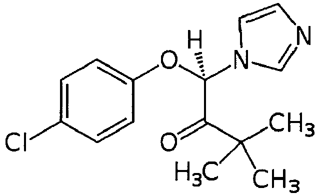

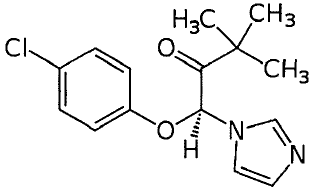

Die exakte Bezeichnung der Verbindung ist (*RS*)-1-(4-Chlorphenoxy)- 1-(1*H*-imidazol-1-yl)- 3,3-dimethylbutan-2-on, der INCI-Name ist Climbazole.

Climbazol wird in bevorzugten erfindungsgemäßen Mitteln innerhalb engerer Mengenbereiche eingesetzt. Erfindungsgemäß bevorzugte Haarbehandlungsmittel sind dadurch gekennzeichnet, daß sie 0,02 bis 7,5, vorzugsweise 0,03 bis 5, weiter bevorzugt 0,04 bis 2,5 und insbesondere 0,05 bis 0,5 Gew.-% Climbazol enthalten.

Die erfindungsgemäßen Mittel können 0,01 bis 10 Gew.-% Zinkpyrithion und/oder Zinkoxid enthalten. Erfindungsgemäße Mittel dieser Ausführungsform enthalten demnach
- 0,01 bis 10 Gew.-% Zinkpyrithion oder
- 0,01 bis 10 Gew.-% Zinkoxid oder
- 0,01 bis 10 Gew.-% einer Mischung aus Zinkpyrithion und Zinkoxid.
Zinkpyrithion 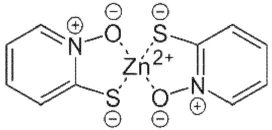 wird chemisch exakt auch als Zink-bis[2-pyridinolat]-*N,N*'-dioxid oder als 2-Pyridinthiol-1-oxid, Zinksalz bezeichnet.

Auch Zinkpyrithion bzw. Zinkoxid wird/werden in bevorzugten erfindungsgemäßen Mitteln innerhalb engerer Mengenbereiche eingesetzt. Erfindungsgemäß bevorzugte Haarbehandlungsmittel sind dadurch gekennzeichnet, daß sie 0,02 bis 7,5, vorzugsweise 0,03 bis 5, weiter bevorzugt 0,04 bis 2,5 und insbesondere 0,05 bis 1 Gew.-% Zinkpyrithion und/oder Zinkoxid enthalten.

Die erfindungsgemäßen Mittel können 0,01 bis 10 Gew.-% Pirocton-Olamin enthalten. Pirocton-Olamin ist eine 1:1 Verbindung von 1-Hydroxy-4-methyl-6-(2,4,4- trimethylpentyl)pyridin-2(1H)-on mit 2-Aminoethanol 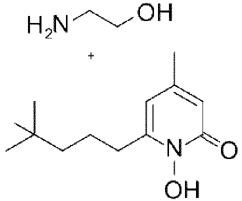 und wird auch als 1-Hydroxy-4-methyl-6-(2,4,4-trimethylpentyl)-2(1*H*)-pyridon monoethanolaminsalz bezeichnet, eine häufig verwendete Handelsbezeichnung ist Octopirox^{®}.

Wenn Pirocton-Olamin in den erfindungsgemäßen Mitteln enthalten ist, liegt dessen Gehalt vorzugsweise zwischen 0,1 und 10 Gew.-%. Auch Pirocton-Olamin wird in bevorzugten erfindungsgemäßen Mitteln innerhalb engerer Mengenbereiche eingesetzt. Erfindungsgemäß bevorzugte Haarbehandlungsmittel sind dadurch gekennzeichnet, daß sie 0,02 bis 7,5, vorzugsweise 0,03 bis 5, weiter bevorzugt 0,04 bis 2,5 und insbesondere 0,05 bis 1 Gew.-% Pirocton-Olamin enthalten.

Die erfindungsgemäßen Mittel können 0,01 bis 10 Gew.-% Selensulfid(e) enthalten. Selensulfid liegt nach der Herstellung über die gängigen Synthesewege als Stoffgemisch vor, das aus Achtringen mit der variablen Zusammensetzung (SeₙS₈₋ₙ) besteht. Aufgrund der typischen Zusammensetzung des Gemischs mit der Verhältnisformel SeS₂ wird häufig auch von *Selendisulfid* gesprochen.

Selensulfide sind aufgrund ihrer fungiziden und gleichzeitig schuppenlösenden Wirkung eine häufige Komponente von Anti-Schuppen-Shampoos. Diese enthalten meist 1% des Wirkstoffs. In höherer Konzentration (2,5%) sind Selensulfide in apothekenpflichtigen Pasten und Suspensionen zur Behandlung des seborrhoischen Ekzems enthalten. Höhere Konzentrationen würden der Arzneimittelverschreibungsverordnung unterliegen.

Wenn Selensulfid(e) in den erfindungsgemäßen Mitteln enthalten ist/sind, liegt dessen/deren Gehalt vorzugsweise zwischen 0,1 und 10 Gew.-%. Auch Selensulfid(e) wird/werden in bevorzugten erfindungsgemäßen Mitteln innerhalb engerer Mengenbereiche eingesetzt. Erfindungsgemäß bevorzugte Haarbehandlungsmittel sind dadurch gekennzeichnet, daß sie 0,02 bis 8, vorzugsweise 0,03 bis 6, weiter bevorzugt 0,04 bis 5 und insbesondere 0,05 bis 3 Gew.-% Selensulfid(e) enthalten.

Die erfindungsgemäßen Mittel können 0,01 bis 10 Gew.-% Salicylsäure enthalten. Die erfindungsgemäßen Mittel können als zusätzlichen Antischuppenwirkstoff Salicylsäure enthalten.

Wenn Salicylsäure in den erfindungsgemäßen Mitteln enthalten ist, liegt deren Gehalt vorzugsweise zwischen 0,1 und 10 Gew.-%. Auch Salicylsäure wird in bevorzugten erfindungsgemäßen Mitteln innerhalb engerer Mengenbereiche eingesetzt. Erfindungsgemäß bevorzugte Haarbehandlungsmittel sind dadurch gekennzeichnet, daß sie zusätzlich 0,01 bis 10 Gew.-%, vorzugsweise 0,02 bis 8, besonders bevorzugt 0,03 bis 6, weiter bevorzugt 0,04 bis 5 und insbesondere 0,05 bis 2 Gew.-% Salicylsäure enthalten.

Ganz besonders bevorzugt werden die Mengen von Epsilon-Poly-L-Lysin(en) und dem Antischuppenwirkstoff b) und dem Antischuppenwirkstoff c) aufeinander abgestimmt, um eine möglichst hohe Effektivität zu bewirken. Hier sind erfindungsgemäße Haarbehandlungsmittel bevorzugt, die eine Gesamtmenge von Epsilon-Poly-L-Lysin der allgemeinen Formel (I) (Inhaltsstoff a)) und Antischuppenwirkstoff b) und Antischuppenwirkstoff c von 0,1 bis 4 Gew.-%, vorzugsweise von 0,25 bis 3,5 Gew.-%, weiter bevorzugt von 0,5 bis 2,5 Gew.-% und insbesondere von 0,6 bis 1,5 Gew.-% enthalten.

Da insbesondere Kopfhautschuppen oft mit einer sehr trockenen Haut einhergehen, ist es bevorzugt, feuchtigkeitsspendende Wirkstoffe in die erfindungsgemäßen Mittel einzuarbeiten. Als besonders verträglich mit der erfindungsgemäßen Kombination haben sich dabei bestimmte Wirkstoffe erwiesen, so daß bevorzugte erfindungsgemäße Haarbehandlungsmittel mindestens einen feuchtigkeitspendenden Aktivstoff aus der Gruppe Pantolacton, Glycerin, Allantoin, Panthenol, Isopentyldiol enthalten.

Unabhängig von der Art des bevorzugten feuchtigkeitsspendenden Wirkstoffs und unabhängig davon, ob lediglich ein solcher Wirkstoff eingesetzt wird oder Mischungen dieser Wirkstoffe zum Einsatz kommen, sind erfindungsgemäße Haarbehandlungsmittel bevorzugt, die den bzw. die feuchtigkeitspendenden Aktivstoff(e) in Mengen von 0,1 bis 10 Gew.-%, vorzugsweise von 0,15 bis 7,5 Gew.-%, weiter bevorzugt von 0,2 bis 5 Gew.-% und insbesondere von 0,25 bis 4 Gew.-% enthalten.

Die kosmetischen Mittel enthalten vorzugsweise weiterhin mindestens einen Vertreter aus der Gruppe der anionischen, amphoteren, zwitterionischen, nichtionischen, kationischen Tenside oder aus Gemischen davon, der wasserunlöslichen Ölkomponenten, der Vitamine, der Provitamine, der Proteinhydrolysate, der Pflanzenextrakte, der UV-Filter, der Aminosäuren, der wasserunlöslichen Silikone, der wasserlöslichen Silikone und/oder der Amodimethicone
Erfindungsgemäß bevorzugte Haarbehandlungsmittel sind dadurch gekennzeichnet, daß sie - bezogen auf ihr Gewicht - 0,5 bis 70 Gew.-%, vorzugsweise 1 bis 60 Gew.-% und insbesondere 5 bis 25 Gew.-% anionische(s) und/oder nichtionische(s) und/oder kationische(s) und/oder amphotere(s) Tensid(e), enthelten.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (T1), 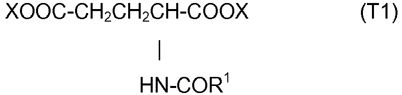 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (T2), 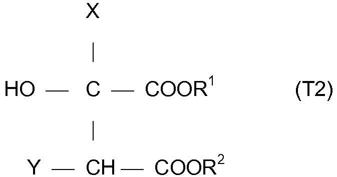 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (T3I), 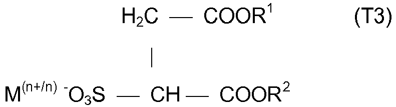 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓOSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethyl-ammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Weiter bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Besonders bevorzugte anionische Tenside sind die Alkali- oder Ammoniumsalze des Laurylethersulfates mit einem Ethoxylierungsgrad von 2 bis 4 EO.

Insbesondere bevorzugte erfindungsgemäße Haarbehandlungsmittel sind dadurch gekennzeichnet, daß sie - bezogen auf ihr Gewicht - 0,1 bis 20 Gew.-%, vorzugsweise 0,25 bis 17,5 Gew.-% und insbesondere 5 bis 15 Gew.-% anionische(s) Tensid(e), besonders bevorzugt Fettalkoholethersulfate der Formel

**H₃C-(CH₂)ₙ-(OCH₂CH₂)ₖ-OSO₃⁻ M⁺**

enthalten, in der n für Werte von 5 bis 21, vorzugsweise von 7 bis 19, besonders bevorzugt von 9 bis 17 und insbesondere von 11 bis 13 und k für Werte von 1, 2, 3, 4, 5, 6, 7, 8, 9 oder 10, vorzugsweise für 1, 2 oder 3 und insbesondere für 2 stehen, und M für ein Kation aus der Gruppe Na⁺, K⁺ NH₄⁺, ½ M²⁺,½Zn²⁺ vorzugsweise für Na⁺, stehen.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethyl-ammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈-C₂₄- Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂-C₁₈- Acylsarcosin.

Besonders bevorzugte erfindungsgemäße Haarbehandlungsmittel sind dadurch gekennzeichnet, daß sie amphotere(s) Tensid(e) aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Kokosalkylaminopropionat, Kokosacylaminoethylaminopropionat, C₁₂ - C₁₈ - Acylsarcosin, N-Alkyl-N,N-dimethylammonium-glycinate, beispielsweise Kokosalkyl-dimethylammoniumglycinat, N-Acyl-aminopropyl-N,N-dimethylammoniumglycinate, beispielsweise Kokosacylaminopropyl-dimethylammoniumglycinat, 2-Alkyl-3-carboxymethyl-3-hydroxyethyl-imidazoline mit jeweils 8 bis 18 C-Atomen in der Alkyl- oder Acylgruppe, Kokosacylaminoethylhydroxyethylcarboxymethylglycinat, der unter der INCI-Bezeichnung Cocamidopropyl Betain bekannten Verbindungen, der unter der INCI-Bezeichnung Disodium Cocoamphodiacetate bekannten Verbindungen enthalten, wobei bevorzugte Mittel das bzw. die amphotere(n) Tensid(e) in Mengen von 0,5 bis 9 Gew.-%, vorzugsweise von 0,75 bis 8 Gew.-% und insbesondere von 1 bis 7,5 Gew.-%, jeweils bezogen auf das gesamte Mittel, enthalten.

Besonders bevorzugte Haarbehandlungsmittel enthalten als amphotere Tenside Betaine der Formel (T4) 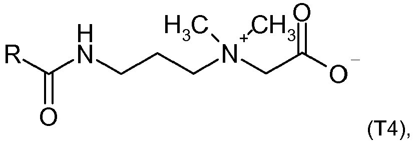 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (T4) eingesetzt, die ein Gemisch der folgenden Vertreter sind:
H₃C-(CH₂)₇-C(O)-NH-(CH₂)₃N⁺(CH₃)₂CH₂COO⁻, H₃C-(CH₂)₉-C(O)-NH-(CH₂)₃N⁺(CH₃)₂CH₂COO⁻, H₃C-(CH₂)₁₁-C(O)-NH-(CH₂)₃N⁺(CH₃)₂CH₂COO⁻, H₃C-(CH₂)₁₃-C(O)-NH-(CH₂)₃N⁺CH₃)₂CH₂COO⁻, H₃C-(CH₂)₁₅-C(O)-NH-(CH₂)₃N⁺(CH₃)₂CH₂COO⁻H₃C-(CH₂)₇-CH=CH-(CH₂)₇-C(O)-NH-(CH₂)₃N⁺(CH₃)₂CH₂COO⁻

Besonders bevorzugt werden Tenside der Formel (T4) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (T4) enthalten.

Zusätzlich zu dem bzw. den Amphotensiden der Formel (T4) oder an deren Stelle können die erfindungsgemäßen Haarbehandlungsmittel mit besonderem Vorzug als amphotere Tenside Betaine der Formel (T5) 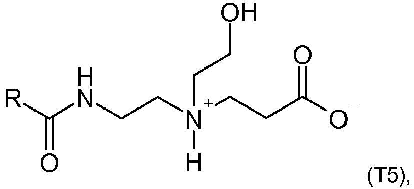 enthalten, in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amphoacetate bezeichnet, wobei die Vertreter, die sich von Kokosfettsäuren ableiten bevorzugt sind und als Cocoamphoactetate bezeichnet werden.

Aus herstellungstechnischen Gründen enthalten Tenside dieses Typs oft auch Betaine der Formel (T5a) 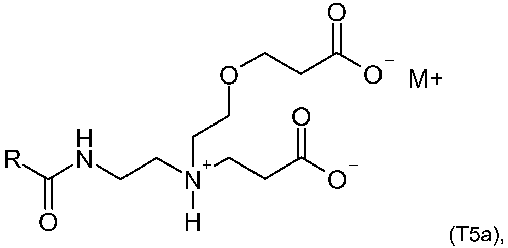 in der R für einen geradkettigen oder verzweigten, gesättigten oder ein- bzw. mehrfach ungesättigten Alkyl- oder Alkenlyrest mit 8 bis 24 Kohlenstoffatomen und M für ein Kation steht. Diese Tenside werden nach der INCI-Nomenklatur als Amphodiacetate bezeichnet, wobei die Vertreter, die sich von Kokosfettsäuren ableiten, bevorzugt sind und als Cocoamphodiactetate bezeichnet werden.

Besonders bevorzugt werden erfindungsgemäß Tenside der Formel (T5) eingesetzt, die ein Gemisch der folgenden Vertreter sind:

H₃C-(CH₂)₇-C(O)-NH-(CH₂)₂NH⁺(CH₂CH₂OH)CH₂CH₂COO⁻

H₃C-(CH₂)₉-C(O)-NH-(CH₂)₂NH⁺(CH₂CH₂OH)CH₂CH₂COO⁻

H₃C-(CH₂)ₙ-C(O)-NH-(CH₂)₂NH⁺(CH₂CH₂OH)CH₂CH₂COO⁻

H₃C-(CH₂)₁₃-C(O)-NH-(CH₂)₂NH⁺(CH₂CH₂OH)CH₂CH₂COO⁻

H₃C-(CH₂)₁₅-C(O)-NH-(CH₂)₂NH⁺(CH₂CH₂OH)CH₂CH₂COO⁻

H₃C-(CH₂)₇-CH=CH-(CH₂)₇-C(O)-NH-(CH₂)₂NH⁺(CH₂CH₂OH)CH₂CH₂COO⁻

Besonders bevorzugt werden Tenside der Formel (T5) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (T5) enthalten.

Zusammenfassend sind erfindungsgemäße Haarbehandlungsmittel bevorzugt, bei denen der Rest R in den Formeln (VI) und (VII) ausgewählt ist aus H₃C-(CH₂)₇-, H₃C-(CH₂)₉-, H₃C-(CH₂)₁₁-, H₃C-(CH₃)₁₃-, H₃C-(CH₂)₁₅-H₃C-(CH₂)₇-CH=CH-(CH₂)₇- oder Mischungen aus diesen.

Besonders bevorzugte nichtionische Tenside sind Alkylpolyglycoside. Demnach sind erfindungsgemäße Haarbehandlungsmittel bevorzugt, die als nichtionische Tenside - bezogen auf ihr Gewicht - 0,1 bis 20 Gew.-% Alkylpolyglycoside der allgemeinen Formel RO-(Z)ₓ enthalten, wobei R für Alkyl, Z für Zucker sowie x für die Anzahl der Zuckereinheiten steht.

Alkylpolyglycoside (APG) sind nichtionische Tenside, die vollständig aus nachwachsenden Rohstoffen (Zuckerbausteine, vorwiegend Glucose z.B. aus Maisstärke und Fettalkohol z.B. aus Kokosöl) hergestellt werden. Alkylpolyglycoside sind durch sauer katalysierte Reaktion (Fischer-Reaktion) von Zuckern, insbesondere Glucose (oder Stärke) oder von Butylglycosiden mit Fettalkoholen zugänglich.

Dabei entstehen komplexe Gemische aus Alkylmonoglucosid (Alkyl-α-D- und -β-D-glucopyranosid sowie geringe Anteile -glucofuranosid), Alkyldiglucosiden (-isomaltoside, -maltoside etc.) und Alkyloligoglucosiden (-maltotrioside, -tetraoside etc.). Der durchschnittliche Polymerisationsgrad kommerzieller Produkte, deren Alkyl-Reste im Bereich C8-C16 liegen, beträgt 1,2-1,5.

Erfindungsgemäß bevorzugt werden Alkylpolyglycoside entsprechend der allgemeinen Formel RO-(Z)ₓ eingesetzt, wobei R für Alkyl, Z für Zucker sowie x für die Anzahl der Zuckereinheiten steht. Besonders bevorzugt sind solche Alkylpolyglycoside, bei denen R im Wesentlichen aus C₈- und C₁₀-Alkylgruppen, oder im wesentlichen aus C₁₂- und C₁₄-Alkylgruppen, oder im wesentlichen aus C₈- bis C₁₆-Alkylgruppen oder oder im wesentlichen aus C₁₂- bis C₁₆-Alkylgruppen oderoder im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.

Weitere Tenside, die - inbesondere in Mischung mit Alkylpolyglycosiden - besonders vorteilhaft in den erfindungsgemäßen Mitteln eingesetzt werden können, sind Glutamate, Asparaginate und Sulfoacetate. Hier sind erfindungsgemäße Haarbehandlungsmittel bevorzugt, die - bezogen auf ihr Gewicht - 0,1 bis 20 Gew.-% Fettsäureglutamate (Acylglutamate) und/oder Fettsäureasparaginate (Acylasparaginate) und/oder Alkylsulfoacetate (Sulfoessigsäure-alkylester) enthalten.

Acylglutamate lassen sich durch die Formel 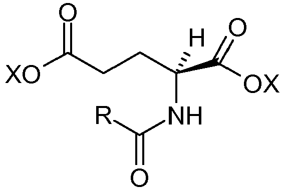 beschreiben, in der R-CO für einen linearen oder verzweigten Acylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alka nolammonium oder Glucammonium steht.

Überraschenderweise wurde gefunden, daß Abmischungen von Alkylglucosiden mit Acylglutamaten eine sehr gute dermatologische Verträglichkeit und ein verbessertes Schaumvermögen sowohl hinsichtlich des Basisschaums als auch der Schaumstabilität in Gegenwart von Wasserhärte aufweisen.

Typische Beispiele für geeignete Acylglutamate sind Aniontenside, die sich von Fettsäuren mit 6 bis 22, vorzugs¬ weise 12 bis 18 Kohlenstoffatomen ableiten, wie beispielsweise C12/14- bzw.

C12/18-Kokosfettsäure, Laurinsäure, Myristinsäure, Palmitinsäure und/oder Stearinsäure. Besonders bevorzugt sind Natrium-N-cocoyl- und Natrium-N-stearoyl-L-glutamat
Die erfindungsgemäßen Mittel können die Alkyl- und/oder Alkenyloligoglucoside und die Acylglutamate im Gewichtsverhältnis 1 : 99 bis 99 : 1, vorzugsweise 10 : 90 bis 90 : 10 und insbesondere 80 : 20 bis 50 : 50 enthalten.

Acylasparaginate lassen sich durch die Formel 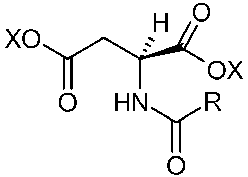 beschreiben, in der R-CO für einen linearen oder verzweigten Acylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.

Typische Beispiele für geeignete Acylasparaginate sind Aniontenside, die sich von Fettsäuren mit 6 bis 22, vorzugsweise 12 bis 18 Kohlenstoffatomen ableiten, wie beispielsweise C12/14- bzw. C12/18-Kokosfettsäure, Laurinsäure, Myristinsäure, Palmitinsäure und/oder Stearinsäure. Besonders bevorzugt sind Natrium-N-cocoyl- und Natrium-N-stearoyl-L-asparaginat

Die erfindungsgemäßen Mittel können die Alkyl- und/oder Alkenyloligoglucoside und die Acylasparaginate ebenfalls im Gewichtsverhältnis 1 : 99 bis 99 : 1, vorzugsweise 10 : 90 bis 90 : 10 und insbesondere 80 : 20 bis 50 : 50 enthalten.

Sulfoacetate (Sulfoessigsäure-ester), sind üblicherweise Salze von Estern der Sulfoessigsäure und lassen sich durch die allgemeine Formel

R-O-C(O)-CH₂-SO₂-OX

beschreiben, in der R für einen linearen oder verzweigten Alkyl- oder Alkenylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.

Besonders bevorzugt ist der Einsatz vom Natriumsalz der Sulfoessigsäure mit der INCI-Bezeichnung: Sodium Lauryl Sulfoacetate):

H₃C-(CH₂)₁₁-O-CO-CH₂-SO₂-ONa

Natriumlaurylsulfoacetat ist ein weißes, freifließendes Pulver, das neutral reagiert, mit gutem Schaumvermögen, Netzvermögen und Dispergiervermögen.

Erfindungsgemäß einsetzbar sind kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Besonders bevorzugte erfindungsgemäße Haarbehandlungsmittel sind dadurch gekennzeichnet, daß sie als kationischen Pflegestoff - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Tensid(e) aus der Gruppe der quartären Ammoniumverbindungen und/oder der Esterquats und/oder der Amidoamine enthalten, wobei bevorzugte kationische(s) Tensid(e) ausgewählt ist/sind aus Alkyltrimethylammoniumchloriden mit vorzugsweise 10 bis 18 C-Atomen im Alkylrest und/oder Dialkyldimethylammoniumchloride mit vorzugsweise 10 bis 18 C-Atomen im Alkylrest und/oder Trialkylmethylammoniumchloride mit vorzugsweise 10 bis 18 C-Atomen im Alkylrest und/oder Cetyltrimethylammoniumchlorid und/oder Stearyltrimethylammoniumchlorid und/oder Distearyldimethylammoniumchlorid und/oder Lauryldimethylammoniumchlorid und/oder Lauryldimethylbenzylammoniumchlorid und/oder Tricetylmethylammoniumchlorid und/oder Quaternium-27 und/oder Quaternium-83 und/oder N-Methyl-N(2-hydroxyethyl)-N,N-(ditalgacyloxyethyl)ammonium-methosulfat und/oder N-Methyl-N(2-hydroxyethyl)-N,N-(distearoyloxyethyl)ammonium-methosulfat und/oder N,N-Dimethyl-N,N-distearoyloxyethyl-ammoniumchlorid und/oder N,N-Di-(2-hydroxyethyl)-N,N-(fettsäureesterethyl)-ammoniumchlorid. Die Pflegeffekte der erfindungsgemäßen Mittel lassen sich noch weiter verstärken, indem bestimmte Pflegestoffe eingesetzt werden. Vorzugsweise werden diese aus bestimmten Gruppen an sich bekannter Pflegestoffe ausgewählt, da diese Pflegestoffe formulierungstechnisch und vom Pflegeffekt hervorragend mit der erfindungsgemäß eingesetzten Kombination gegen Schuppen harmonieren.

Erfindungsgemäß bevorzugte Haarbehandlungsmittel sind dadurch gekennzeichnet, daß sie zusätzlich Pflegestoff(e) - bezogen auf ihr Gewicht - in Mengen von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% enthalten, wobei bevorzugte Pflegstoff(e) ausgewählt sind aus der Gruppe
i. L-Carnitin und/oder seiner Salze;
ii. Taurin und/oder seiner Salze;
iii. Niacinamid;
iv. Ubichinon
v. Ectoin;

Erfindungsgemäße bevorzugte Haarbehandlungsmittel sind dadurch gekennzeichnet, daß sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Ein weiterer, bevorzugter einsetzbarer Pflegestoff, der aktivierende Eigenschaften besitzt, ist das Taurin. Erfindungsgemäß bevorzugte Haarbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflegestoffen in den erfindungsgemäßen Mitteln sind Vitamine, Provitamine oder Vitaminvorstufen. Diese werden nachfolgend beschrieben:
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt (siehe weiter unten). Erfindungsgemäß einsetzbare Derivate des Panthenols sind insbesondere die Ester und Ether des Panthenols sowie kationisch derivatisierte Panthenole. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Haarbehandlungsmittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-N-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß bestimmte Chinone eine besondere Eignung als Pflegestoff besitzen. Als weiteren Pflegestoff können die erfindungsgemäßen Mittel daher 0,0001 bis 5 Gew.-% mindestens eines Biochinons der Formel (Ubi) 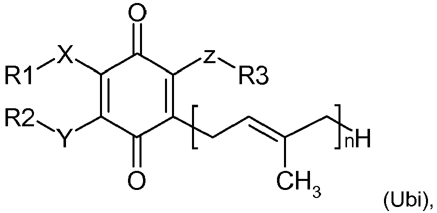 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H,-CH₃, -CH₂CH₃, -(CH₂)₂CH₂, -CH(CH₃)₂, -(CH₂)₃CH₃, -CH(CH₃)CH₂CH₃,-CH₂CH(CH₃)₂, -C(CH₃)₃
- R⁴: steht für -CH₃, -CH₂CH₃, -(CH₂)₂CH₂, -CH(CH₃)₂,-(CH₂)₃CH₃, -CH(CH₃)CH₂CH₃, -CH₂CH(CH₃)₂, -C(CH₃)₃
- n: steht für Werte von 1 bis 20, vorzugsweise von 2 bis 15 und insbesondere für 5, 6, 7, 8, 9, 10.

Besonders bevorzugte erfindungsgemäße Haarbehandlungsmittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 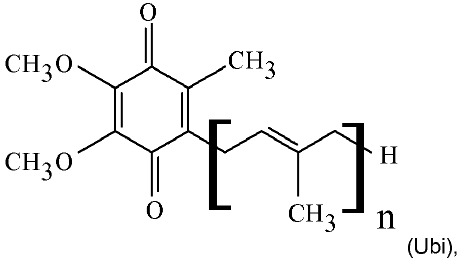 in der n für die Werte = 6, 7, 8, 9 oder 10, besonders bevorzugt für 10 (Coenzym Q10) steht.

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, daß sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-b) enthalten 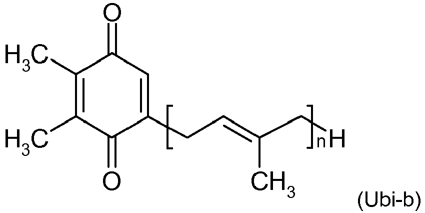 in der n für Werte von 1 bis 20, vorzugsweise von 2 bis 15 und insbesondere für 5, 6, 7, 8, 9, 10 steht, wobei besonders bevorzugt Mittel Plastochinon PQ-9 der Formel 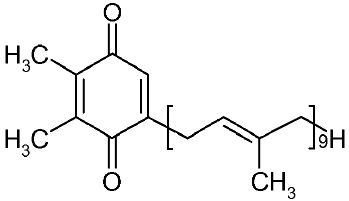 enthalten.

Als weiteren Pflege-Enhacer können die erfindungsgemäßen Mittel Ectoin enthalten. Ectoin ((4S)-2-Methyl-1,4,5,6-Tetrahydropyrimidin-4-Carbonsäure) ist ein zur Gruppe der kompatiblen Solute gehörender Naturstoff. Die stark wasserbindende niedermolekulare organische Verbindung tritt in halophilen Bakterien auf und ermöglicht diesen extremophilen Organismen unter Stressbedingungen zu überleben. Erfindungsgemäß bevorzugte Haarbehandlungsmittel sind dadurch gekennzeichnet, daß sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Zur Verbesserung der Elastizität und Festigung der inneren Struktur der mit erfindungsgemäßen Mitteln behandelter Haare können die erfindungsgemäßen Mittel Purin und/oder Purinderivate als Pflegestoff enthalten. Insbesondere die Kombination von Purin und/oder Purinderivaten mit Ubichinonen und/oder Plastochinonen als Pflegestoff führt dazu, daß die mit entsprechenden Mitteln behandelten Haare unter anderem höhere Meßwerte bei der Differenzthermoanalyse und verbesserte Naß- und Trockenkämmbarkeiten zeigen.

Bevorzugte erfindungsgemäße Mittel enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Erfindungsgemäß bevorzugte Haarbehandlungsmittel sind dadurch gekennzeichnet, daß sie als Pflegestoff - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 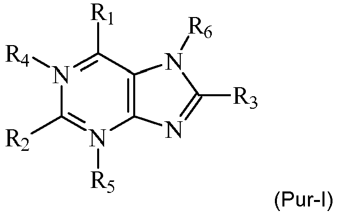 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Es ist weiterhin vorteilhaft, Purin bzw. Purinderivate und Biochinone in einem bestimmten Verhältnis zueinander einzusetzen. Hier sind erfindungsgemäße Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.

Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflegestoff können die erfindungsgemäßen Mittel auch Flavonoide enthalten. Die Flavonoide sind eine Gruppe von wasserlöslichen Pflanzenfarbstoffen und spielen eine wichtige Rolle im Stoffwechsel vieler Pflanzen. Sie gehören zusammen mit den Phenolsäuren zu den Polyphenolen. Es sind weit über 6500 unterschiedliche Flavonoide bekannt, die sich in Flavonole, Flavone, Flavanone, Isoflavonoide und Anthocyane einteilen lassen.

Erfindungsgemäß können Flavonoide aus allen sechs Gruppen eingesetzt werden, wobei bestimmte Vertreter aus den einzelnen Gruppen als Pflegestoff wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Haarbehandlungsmittel sind dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-*O*-rutinosid (Rutin), enthalten.

Bevorzugt ist auch der Einsatz von Bisabolol und/oder Bisabololoxiden als Pflegestoff in den erfindungsgemäßen Mitteln. Hier sind erfindungsgemäße Haarbehandlungsmittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Auch Creatin eignet sich erfindungsgemäß als Pflegestoff. Creatin (3-Methylguanidinoessigsäure) ist eine organische Säure, die in Wirbeltieren u. a. zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab und ist zu 95 % im Skelettmuskel vorhanden. Besonders bevorzugte erfindungsgemäße Haarbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% N-Methylguanidino-essigsäure (Creatin).

Die erfindungsgemäßen Mittel können zusätzlich zu den vorstehend genannten Inhaltsstoffen und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben:
Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, daß sich weniger DHT aus Testosteron bilden kann.

Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich.

Zur Bekämpfung der hormonellen Einflüsse auf die Haarfollikel kann zur äußeren Anwendung Spironolactone in Form von Haarwasser und in Kombination mit Minoxidil angewandt werden. Spironolactone wirkt als Androgen-Rezeptor-Blocker, dh. die Bindung von DHT an die Haarfollikel wird verhindert.

Zusammenfassend sind erfindungsgemäße Haarbehandlungsmittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten.

Zusätzlich zu den Pflegestoffen können di erfindungsgemäßen Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, *S*-Methyl-L-methionin, *S*-Allyl-L-cystein-sulfoxid (L-Alliin), L*-trans-*4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Haarbehandlungsmittel dadurch gekennzeichnet, daß sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel dadurch gekennzeichnet, daß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose, Disacchariden, insbesondere Saccharose und/oder Maltose und/oder Lactose und/oder Trehalose und/oder Cellobiose und/oder Gentiobiose und/oder Isomaltose.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.

Wie bereits erwähnt, enthalten bevorzugte erfindungsgemäße Mittel (eine) Aminosäure(n).

Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte kosmetische Mittel dadurch gekennzeichnet, daß sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat und 0,1 bis 0,25 Gew.-% Glycin,
- 0,005 bis 0,015 Gew.-% Coffein und 0,75 bis 1,5 Gew.-% Saccharose und 0,1 bis 0,25 Gew.-% Glycin,
- 0,005 bis 0,015 Gew.-% Coffein und 0,75 bis 1,5 Gew.-% Fructose und 0,1 bis 0,25 Gew.-% Glycin,
- 0,005 bis 0,015 Gew.-% Coffein und 0,75 bis 1,5 Gew.-% Glucosemonohydrat und 0,1 bis 0,25 Gew.-% Alanin,
- 0,005 bis 0,015 Gew.-% Coffein und 0,75 bis 1,5 Gew.-% Saccharose und 0,1 bis 0,25 Gew.-% Alanin,
- 0,005 bis 0,015 Gew.-% Coffein und 0,75 bis 1,5 Gew.-% Fructose und 0,1 bis 0,25 Gew.-% Alanin,
- 0,005 bis 0,015 Gew.-% Coffein und 0,75 bis 1,5 Gew.-% Glucosemonohydrat und 0,1 bis 0,25 Gew.-% Valin,
- 0,005 bis 0,015 Gew.-% Coffein und 0,75 bis 1,5 Gew.-% Saccharose und 0,1 bis 0,25 Gew.-% Valin,
- 0,005 bis 0,015 Gew.-% Coffein und 0,75 bis 1,5 Gew.-% Fructose und 0,1 bis 0,25 Gew.-% Valin.

Eine besonders bevorzugte Gruppe von Inhaltsstoffen stellen die Silikone dar.

Erfindungsgemäß besonders bevorzugte Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Bevorzugte Silikone werden nachstehend beschrieben.

Besonders bevorzugte erfindungsgemäße Mittel sind dadurch gekennzeichnet, daß sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Diese Silikone werden nach der INCI-Nomenklatur als DIMETHICONE bezeichnet. Es werden im Rahmen der vorliegenden Erfindung als Silicon der Formel Si-I vorzugsweise die Verbindungen: (CH₃)₃Si-O-Si(CH₃)₃, (CH₃)₃Si-O-(CH₃)₂Si-O-Si(CH₃)₃, (CH₃)₃Si-[O-(CH₃)₂Si]₂-O-Si(CH₃)₃, (CH₃)₃Si-[O-(CH₃)₂Si]₃-O-Si(CH₃)₃, (CH₃)₃Si-[O-(CH₃)₂Si]₄-O-Si(CH₃)₃, (CH₃)₃Si-[O-(CH₃)₂Si]₅-O-Si(CH₃)₃, (CH₃)₃Si-[O-(CH₃)₂Si]₆-O-Si(CH₃)₃, (CH₃)₃Si-[O-(CH₃)₂Si]₇-O-Si(CH₃)₃, (CH₃)₃Si-[O-(CH₃)₂Si]₈-O-Si(CH₃)₃, (CH₃)₃Si-[O-(CH₃)₂Si]₉-O-Si(CH₃)₃, (CH₃)₃Si-[O-(CH₃)₂Si]₁₀-O-Si(CH₃)₃, (CH₃)₃Si-[O-(CH₃)₂Si]₁₁-O-Si(CH₃)₃, (CH₃)₃Si-[O-(CH₃)₂Si]₁₂-O-Si(CH₃)₃, (CH₃)₃Si-[O-(CH₃)₂Si]₁₃-O-Si(CH₃)₃, (CH₃)₃Si-[O-(CH₃)₂Si]₁₄-O-Si(CH₃)₃, (CH₃)₃Si-[O-(CH₃)₂Si]₁₅-O-Si(CH₃)₃, (CH₃)₃Si-[O-(CH₃)₂Si]₁₆-O-Si(CH₃)₃, (CH₃)₃Si-[O-(CH₃)₂Si]₁₇-O-Si(CH₃)₃, (CH₃)₃Si-[O-(CH₃)₂Si]₁₈-O-Si(CH₃)₃, (CH₃)₃Si-[O-(CH₃)₂Si]₁₉-O-Si(CH₃)₃, (CH₃)₃Si-[O-(CH₃)₂Si]₂₀-O-Si(CH₃)₃ eingesetzt, wobei (CH₃)₃Si--O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind. Selbstverständlich können auch Mischungen der o.g. Silikone in den erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind. Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2)x}(R_{c}SiO_{(4-c)/2)y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-,-OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist-N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind dadurch gekennzeichnet, daß sie ein aminofunktionelles Silikon der Formel (Si-II)

R'ₐG₃₋ₐ-Si(OSiG₂)ₙ-(OSiG_{b}R'_{2-b})ₘ-O-SiG₃₋ₐ-R'ₐ (Si-II),

enthalten, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃,-CH₂CH₂CH₂CH₃, -O-CH₂CH(CH₃)₂, -CH₂CH(CH₃)₂, -O-CH(CH₃)CH₂CH₃,-CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A⁻
   ∘ -Q-N⁺H₂(R")A⁻
   ∘ -Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie mindestens ein aminofunktionelles Silikon der Formel (Si-IIa) 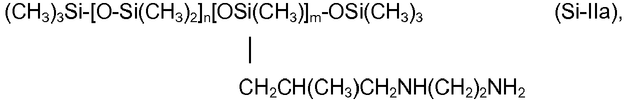 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet. Besonders bevorzugt sind auch erfindungsgemäße Mittel, die ein aminofunktionelles Silikon der Formel (Si-IIb) 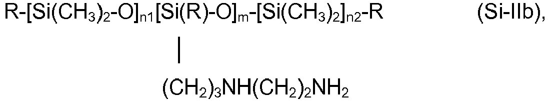 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Erfindungsgemäß bevorzugte Mittel sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße Mittel bevorzugt, die mindestens ein Silikon der Formel Si-III 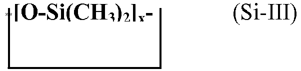 enthalten, in der x für eine Zahl von 3 bis 200, vorzugsweise von 3 bis 10, weiter bevorzugt von30 bis 7 und insbesondere 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Erfindungsgemäß ebenfalls bevorzugte Mittel sind dadurch gekennzeichnet, daß sie mindestens ein Silikon der Formel Si-IV

R₃Si-[O-SiR₂]ₓ-(CH₂)ₙ-[O-SiR₂]_{y}-O-SiR₃ (Si-IV),

enthalten, in der R für gleiche oder verschiedene Reste aus der Gruppe -H, - Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Mittel sind dadurch gekennzeichnet, daß sie mindestens ein wasserlösliches Silikon enthalten.

Aus ästhetischen Gründen werden "klare" Produkte von Verbrauchern oft bevorzugt. Erfindungsgemäß bevorzugte Haarbehandlungsmittel sind daher dadurch gekennzeichnet, daß sie transparent bzw. transluzent sind.

Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert;* NTU) ist eine in der Wasseraufbereitung verwendete Einheit für Trübungsmessungen in Flüssigkeiten. Sie ist die Einheit einer mit einem kalibriertem Nephelometer gemessenen Trübung einer Flüssigkeit.

Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4`-dimethoxybenzophenon (Benzophenone-6), 2,2'-Dihydroxy-4,4'-dimethoxybenzophenon-5-natriumsulfonat und 2-Cyano-3,3-diphenylacrylsäure-2`-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2`-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4`-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Die UV-Filter (I) sind in den erfindungsgemäßen Mitteln üblicherweise in Mengen 0,1-5 Gew.-%, bezogen auf das gesamte Mittel, enthalten. Mengen von 0,4-2,5 Gew.-% sind bevorzugt.

Die erfindungsgemäßen Mittel können weiterhin eine 2-Pyrrolidinon-5-carbonsäure und deren Derivate (J) enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn zusätzlich zu dem bzw. den Polymer(en) aus der Gruppe der kationischen und/oder amphoteren Polymere weitere Polymere (G) in den erfindungsgemäßen Mitteln enthalten sind. In einer bevorzugten Ausführungsform werden den erfindungsgemäßen Mitteln daher weitere Polymere zugesetzt, wobei sich sowohl anionische als auch nichtionische Polymere als wirksam erwiesen haben.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere
- Celluloseether, wie Hydroxypropylcellulose, Hydroxyethylcellulose und Methylhy-droxypropylcellulose,
- Stärke und deren Derivate, insbesondere Stärkeether
- Schellack
- Polyvinylpyrrolidone,
- Siloxane. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten.

Die weiteren Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Ein erfindungsgemäßes Haarbehandlungsmittel wird auf keratinische Fasern aufgebracht und dort entweder bis zur nächsten Haarwäsche belassen (sogenanntes "leave-on"-Produkt) oder nach einer Einwirkzeit von 30 bis 300 Sekunden ausgespült (sogenanntes "rinse-off'-Produkt) wird.

Dieses nicht-therapeutische kosmetische Verfahren führt zu einer Verringerung der Schuppenbildung. Beispiele (Alle Angaben in Gew.-%.):

### 1. Haartonics:

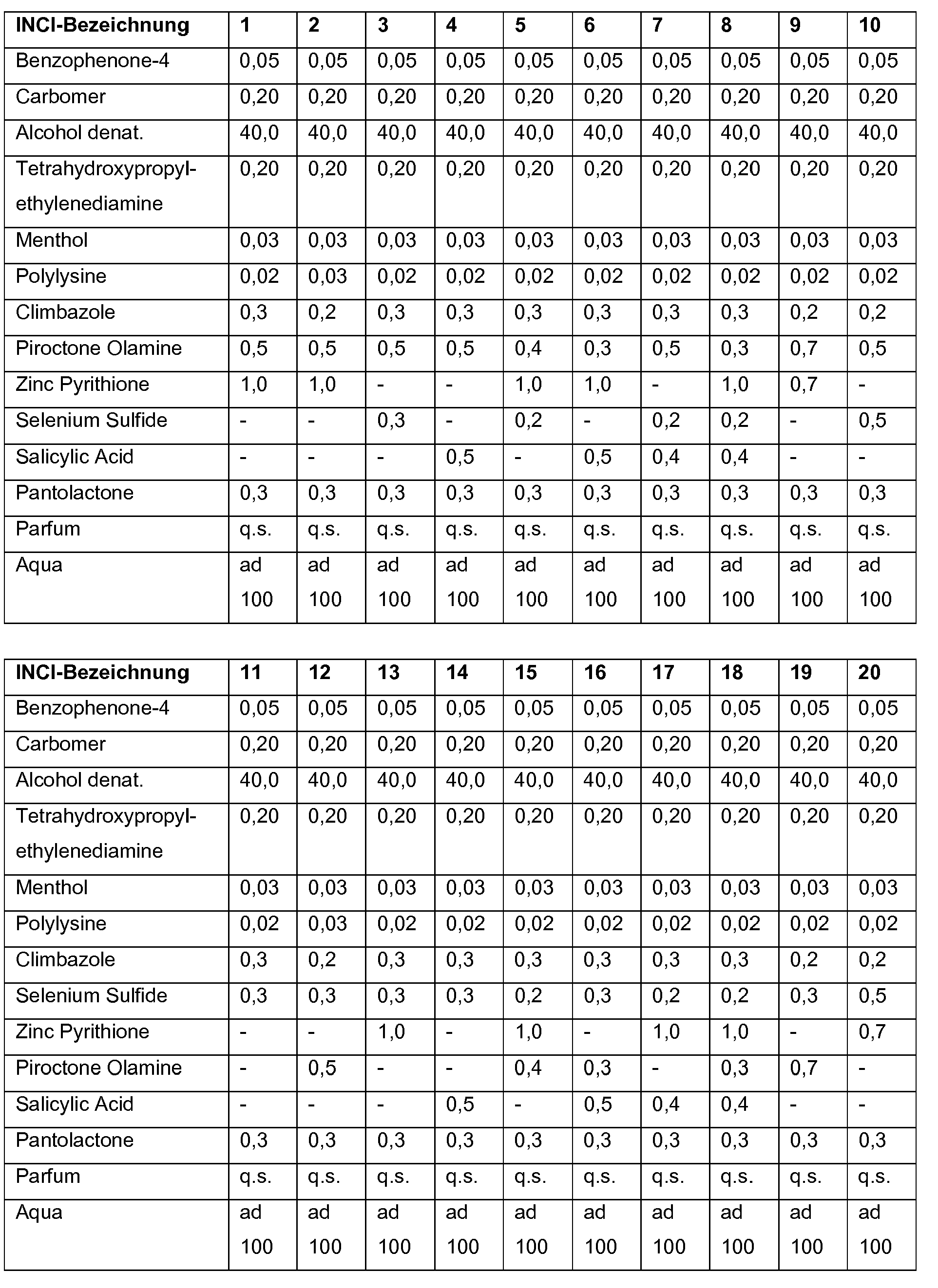

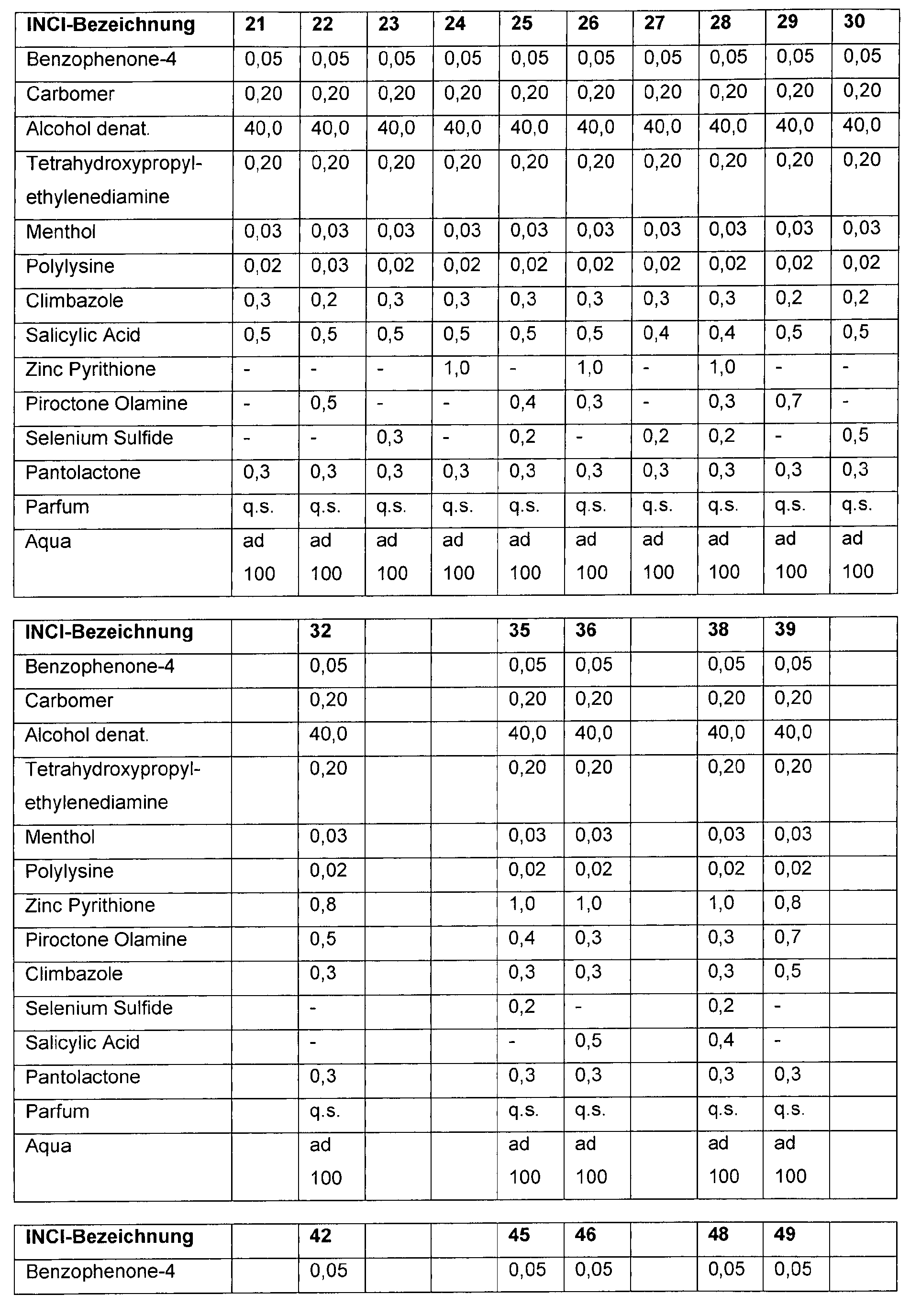

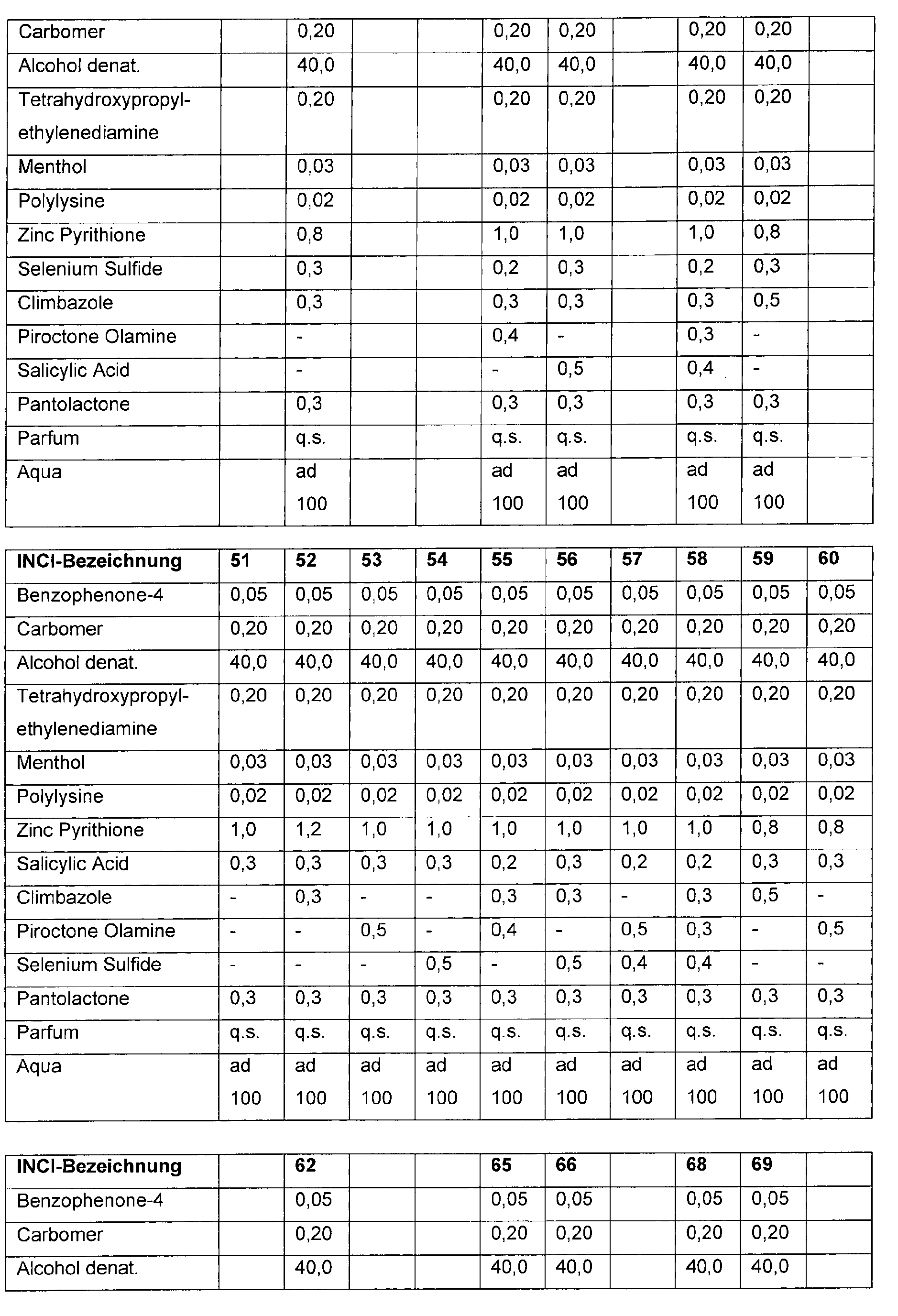

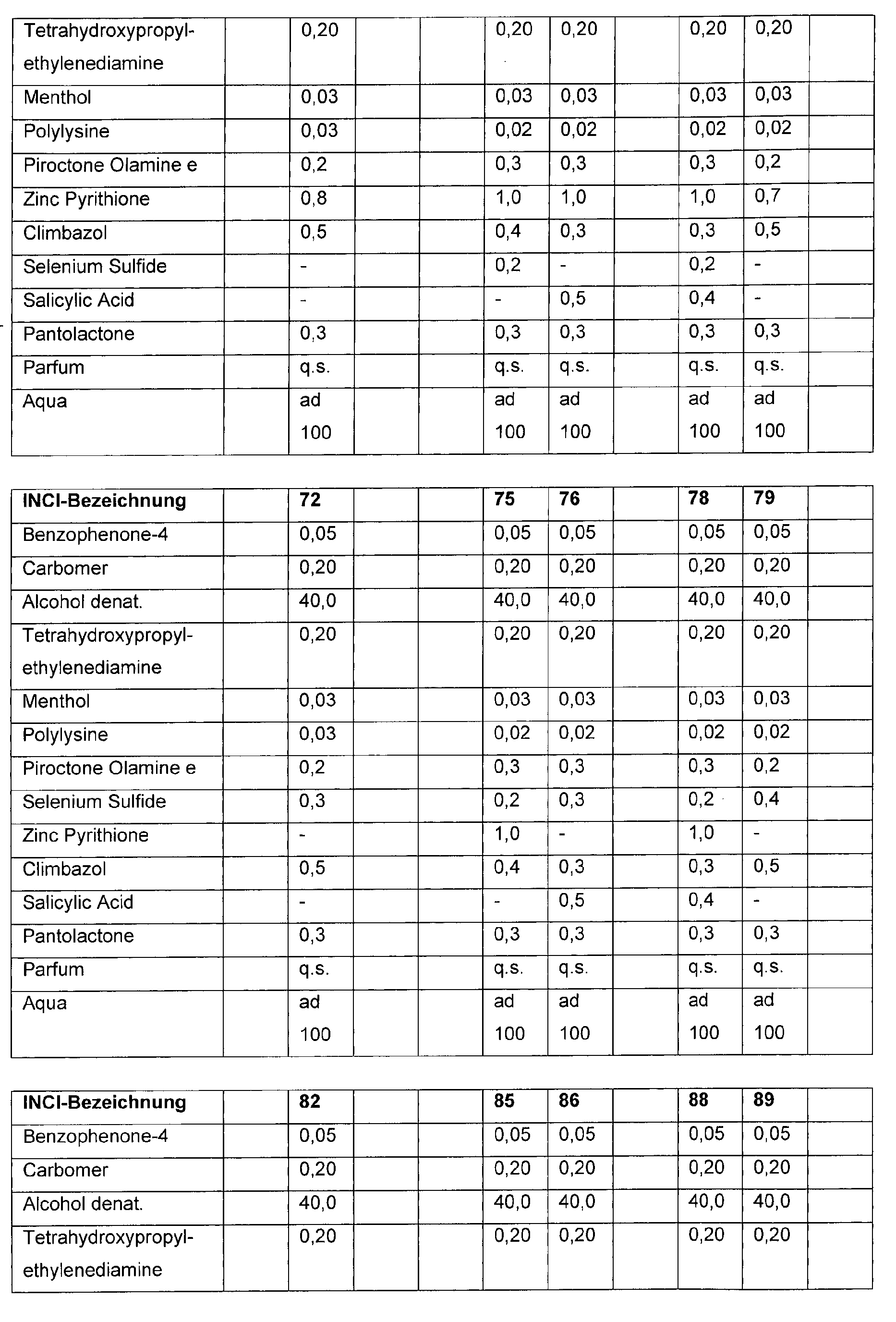

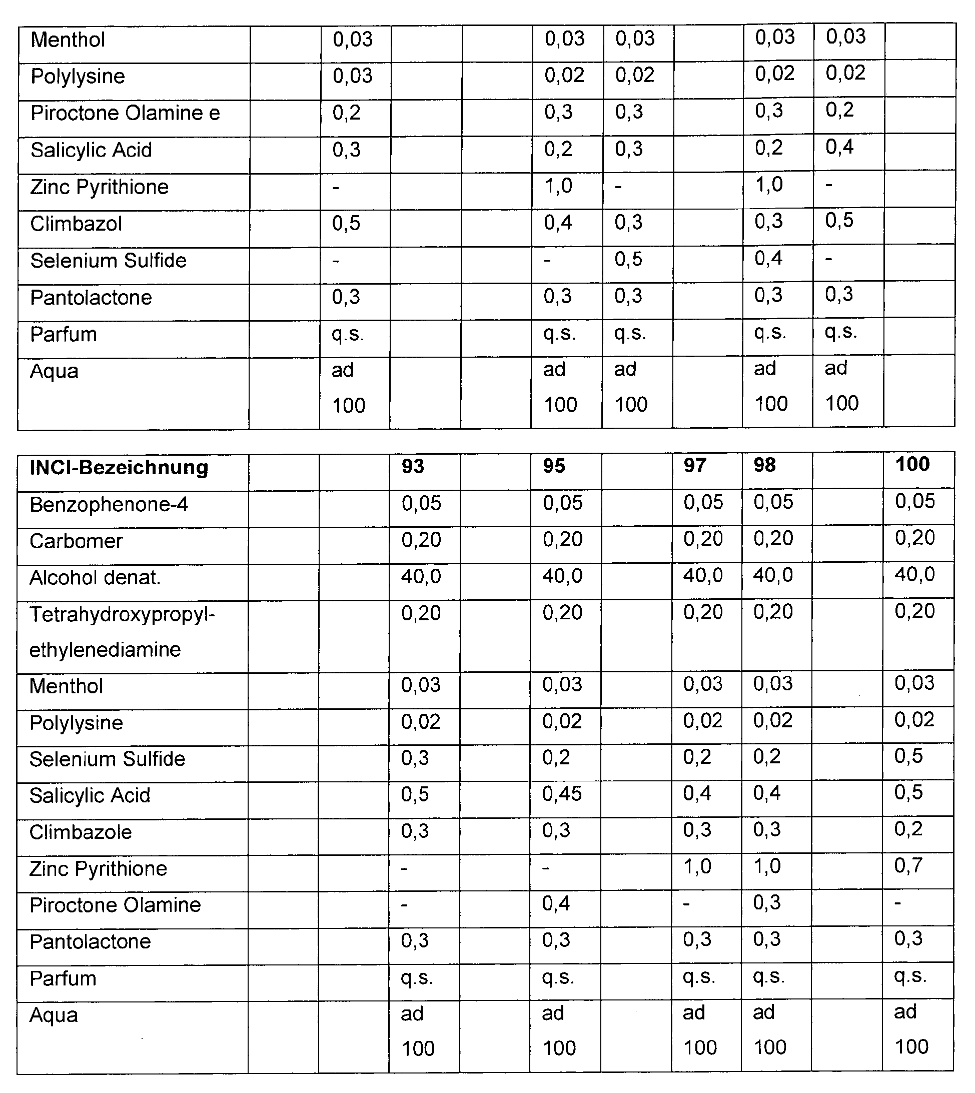

### 1. Haarspülungen:

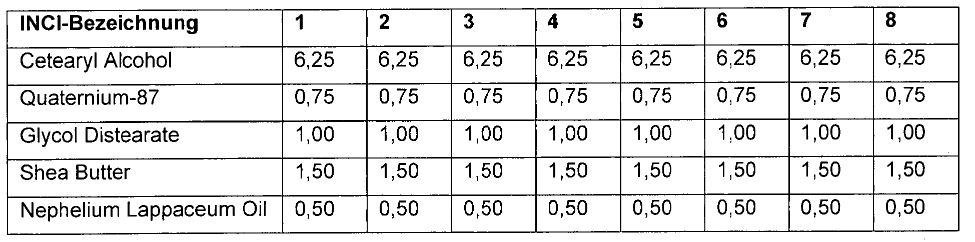

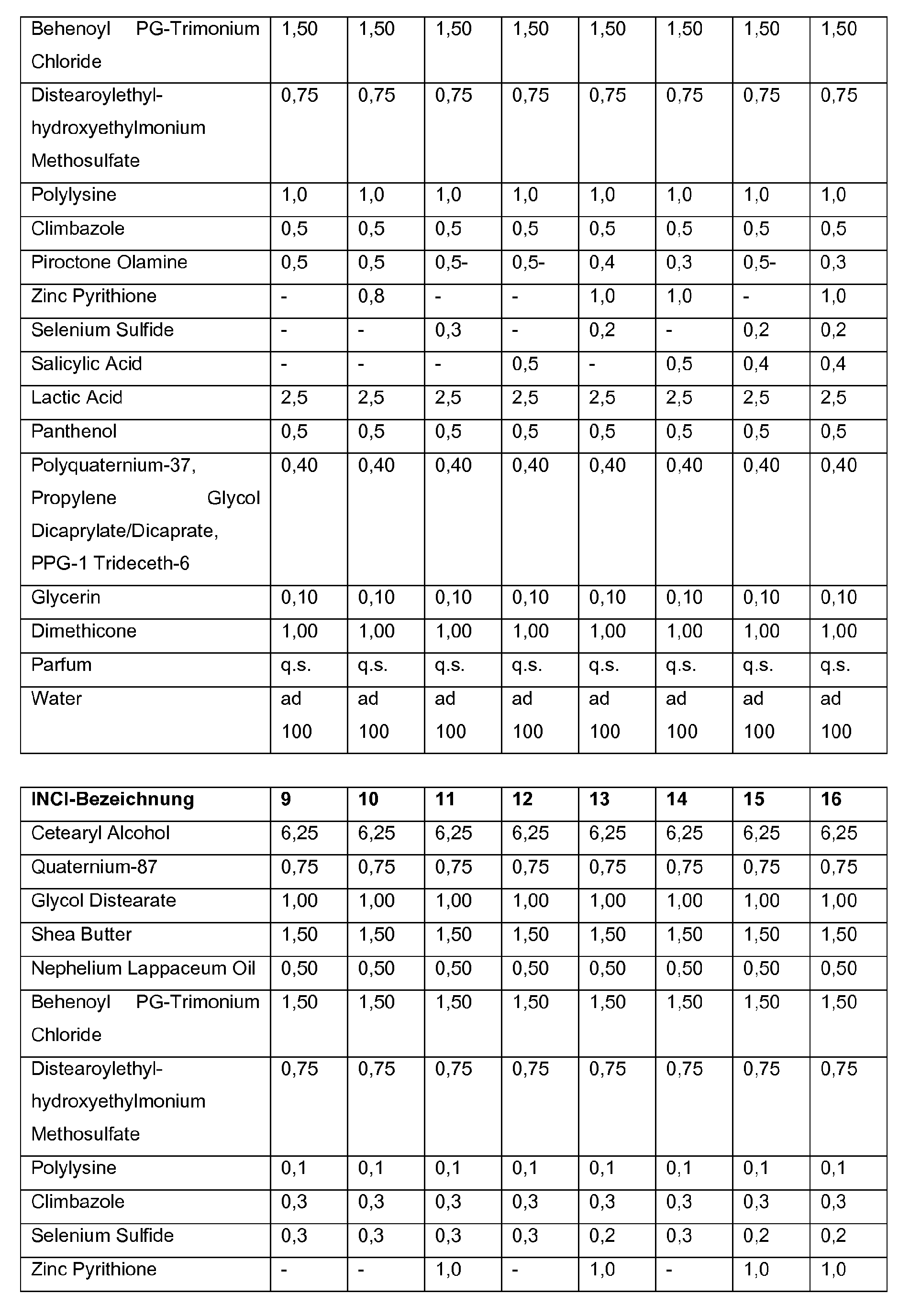

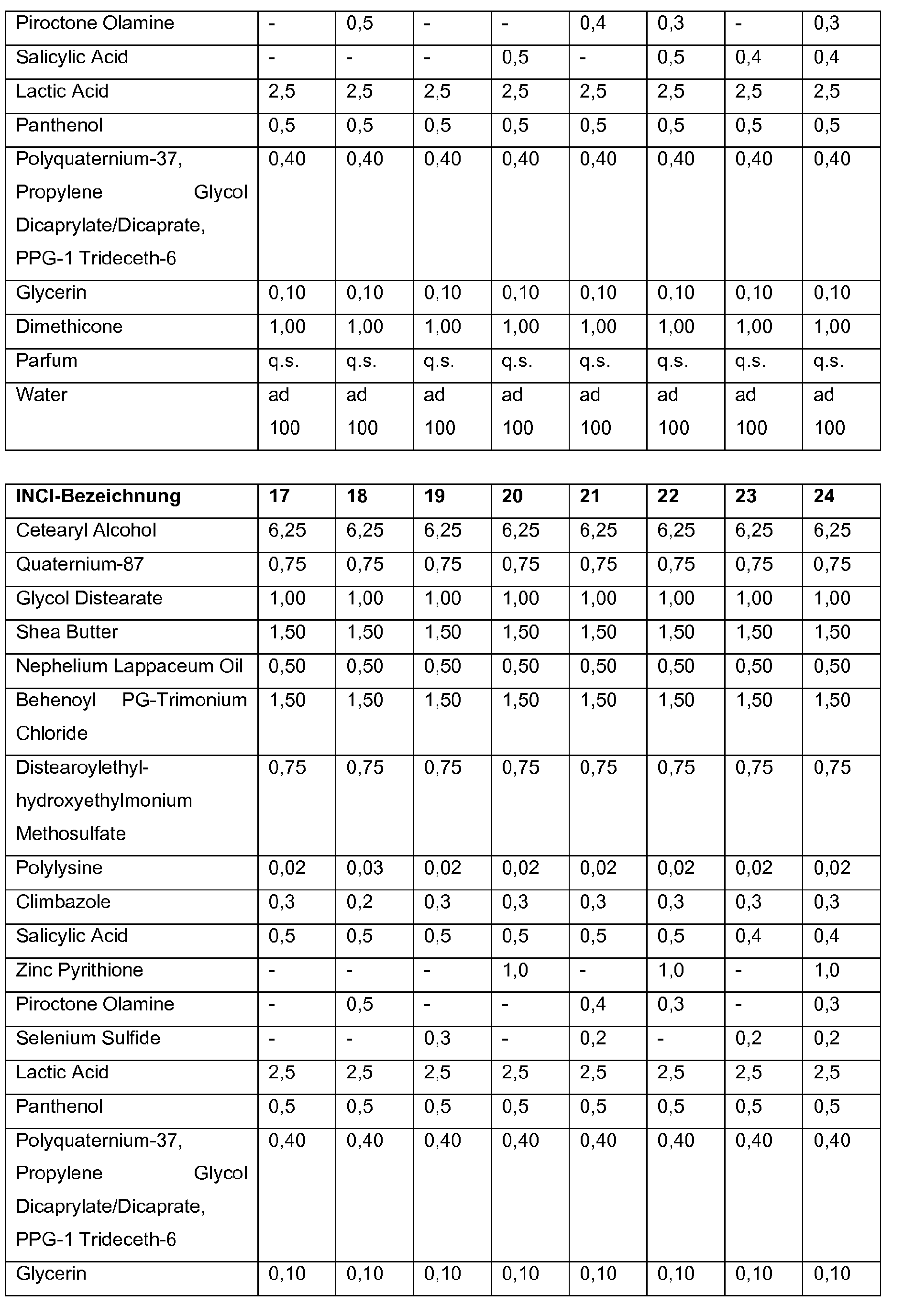

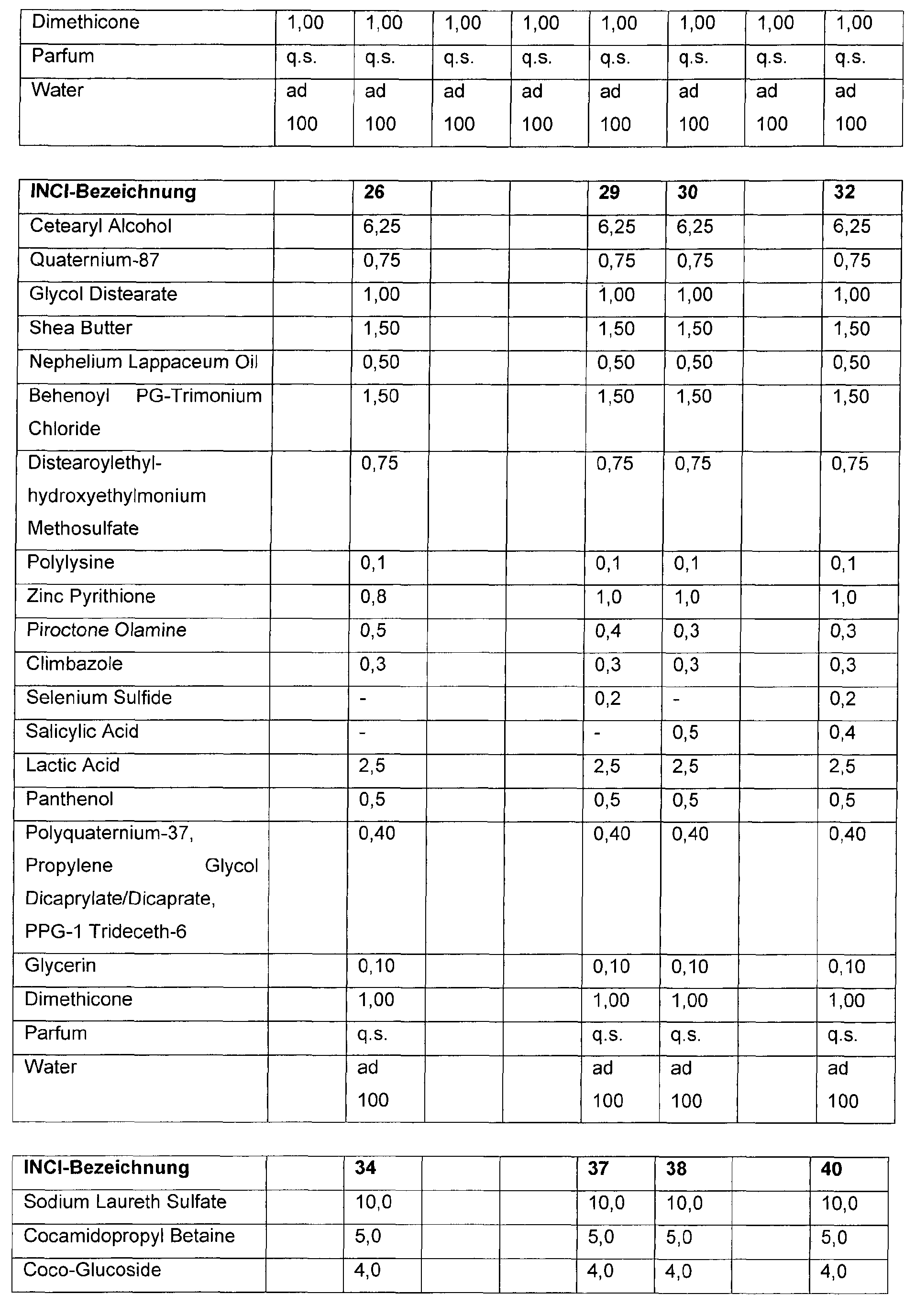

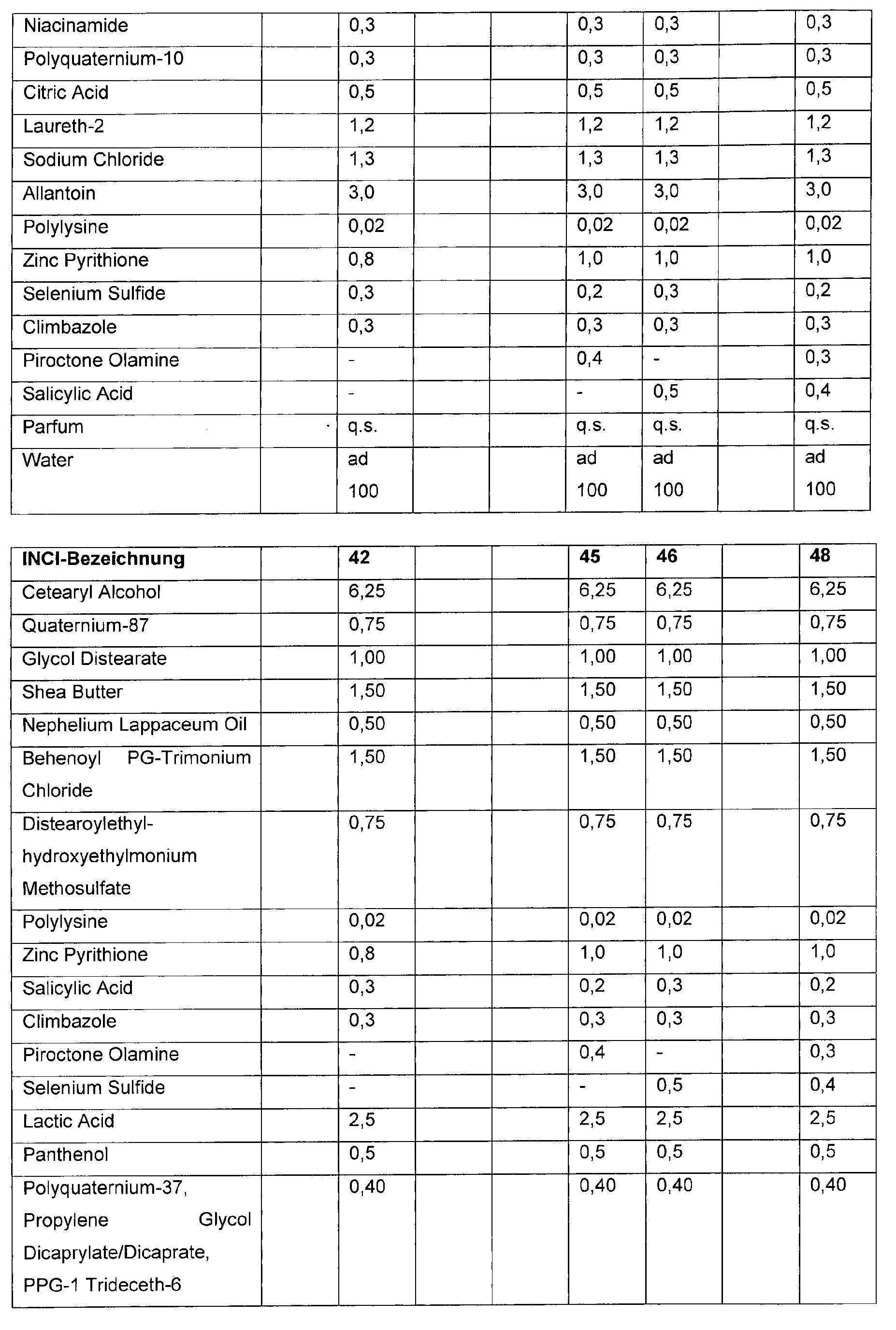

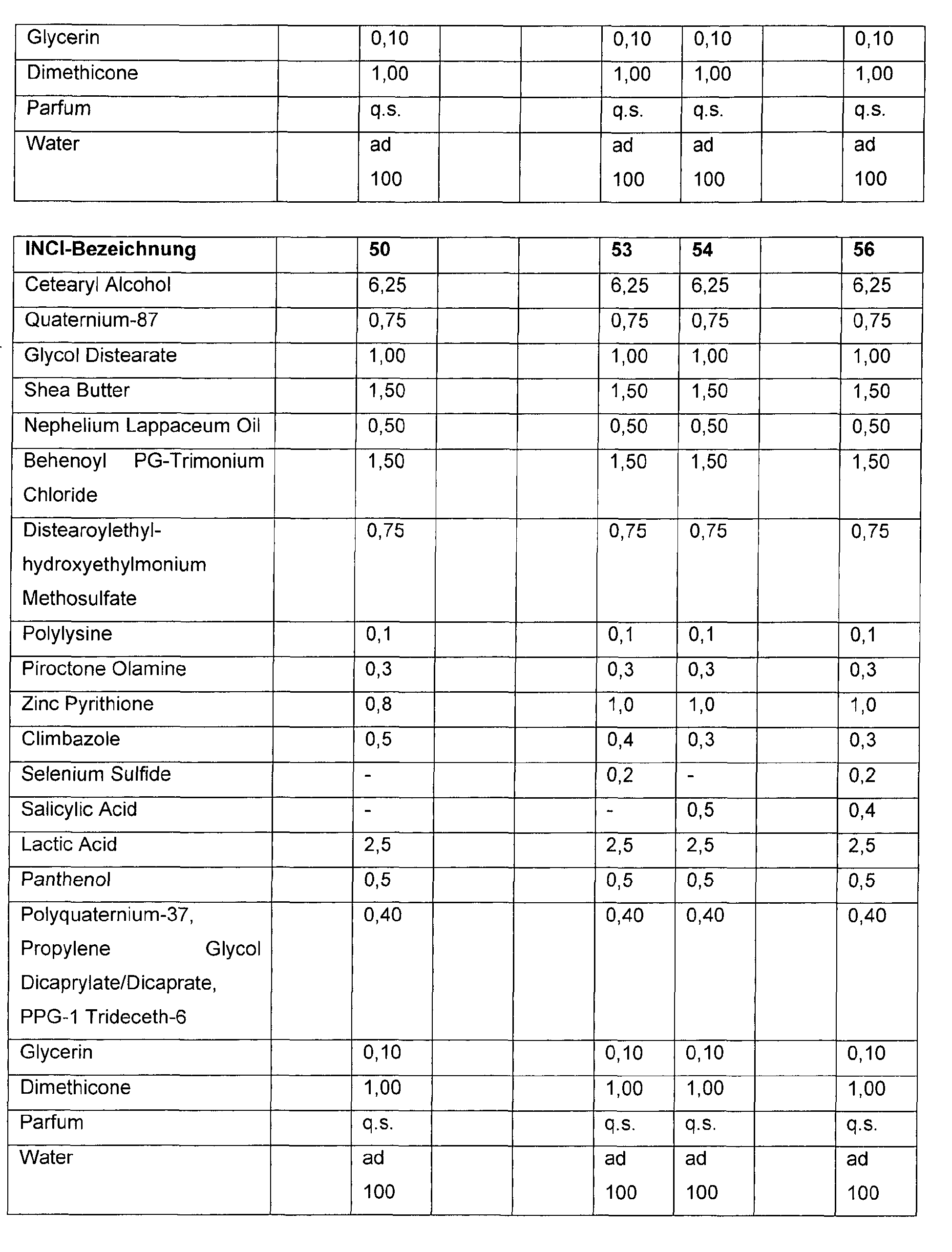

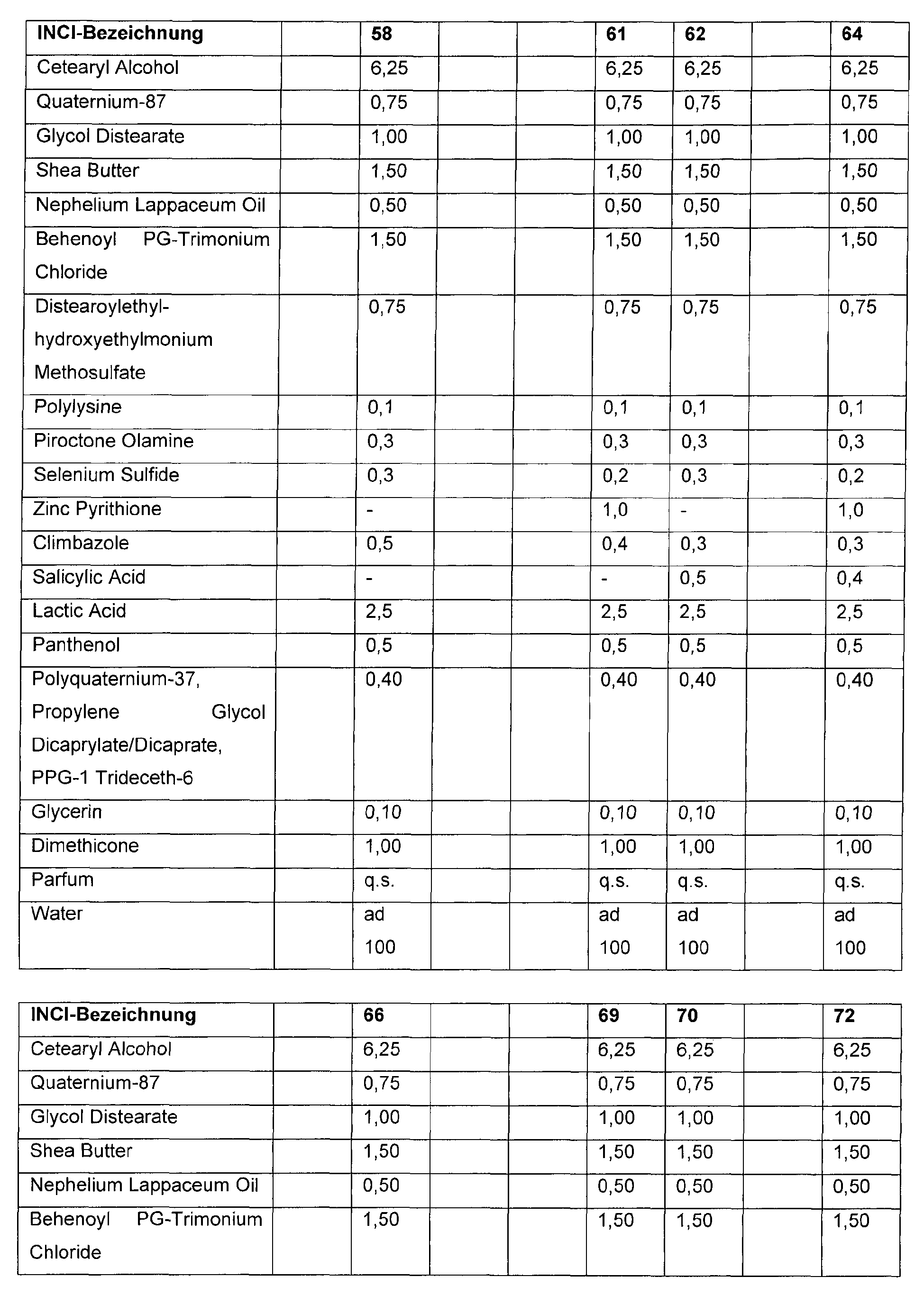

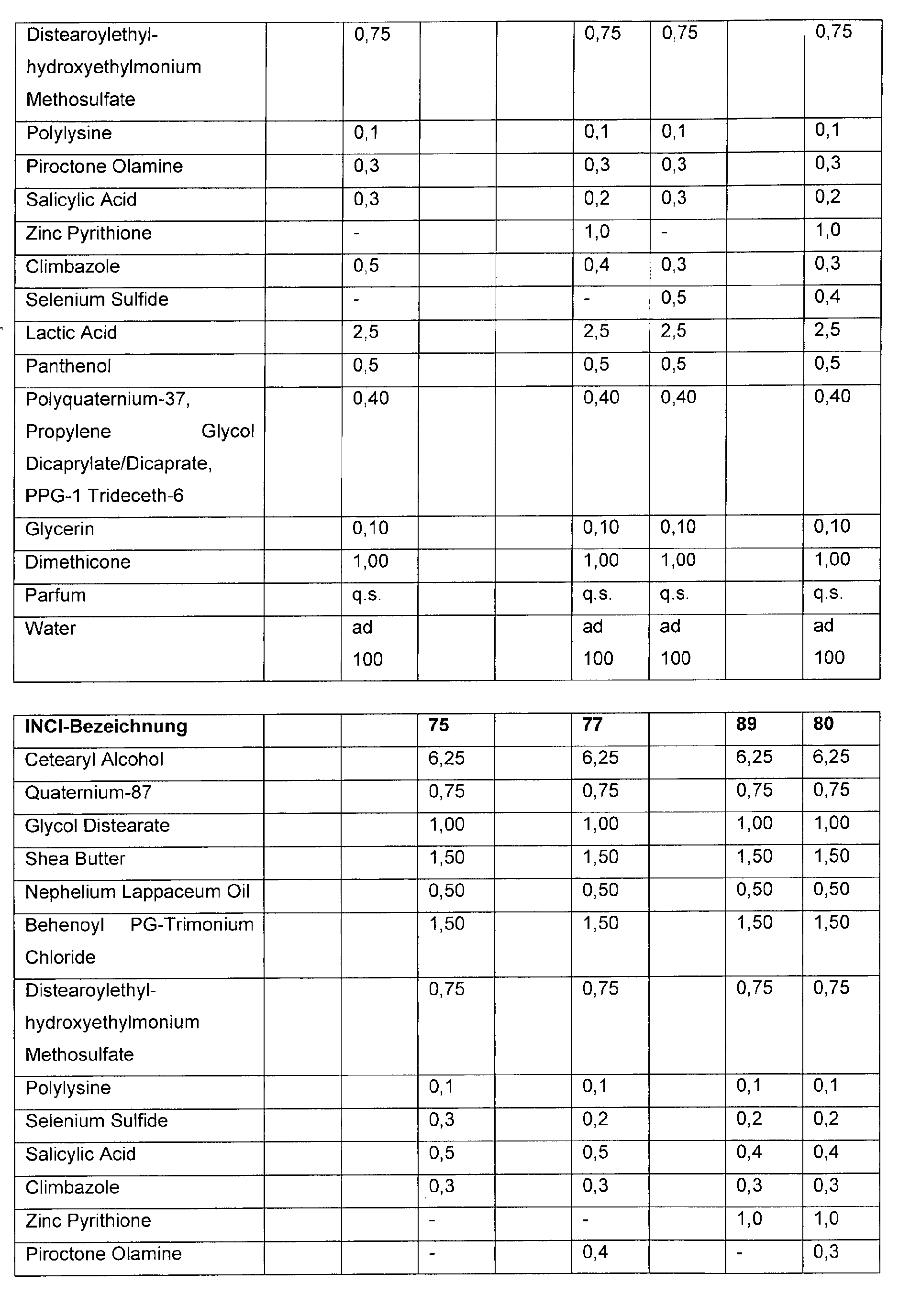

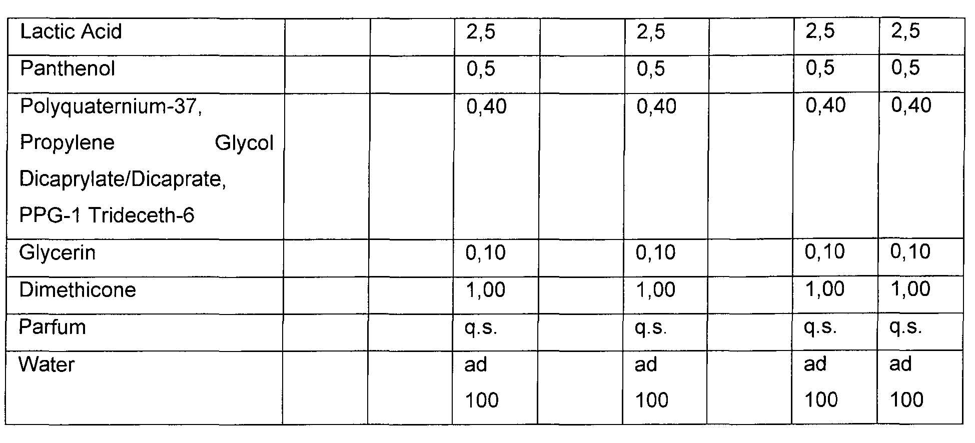

### 2. Haarshampoos:

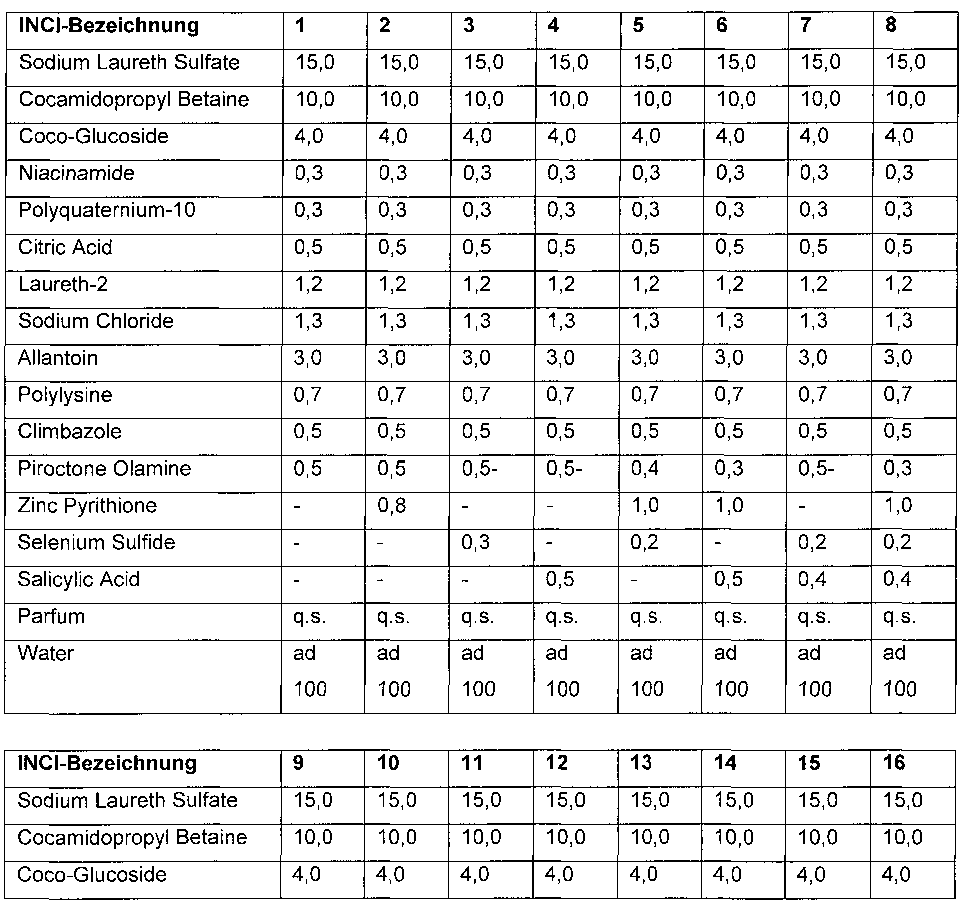

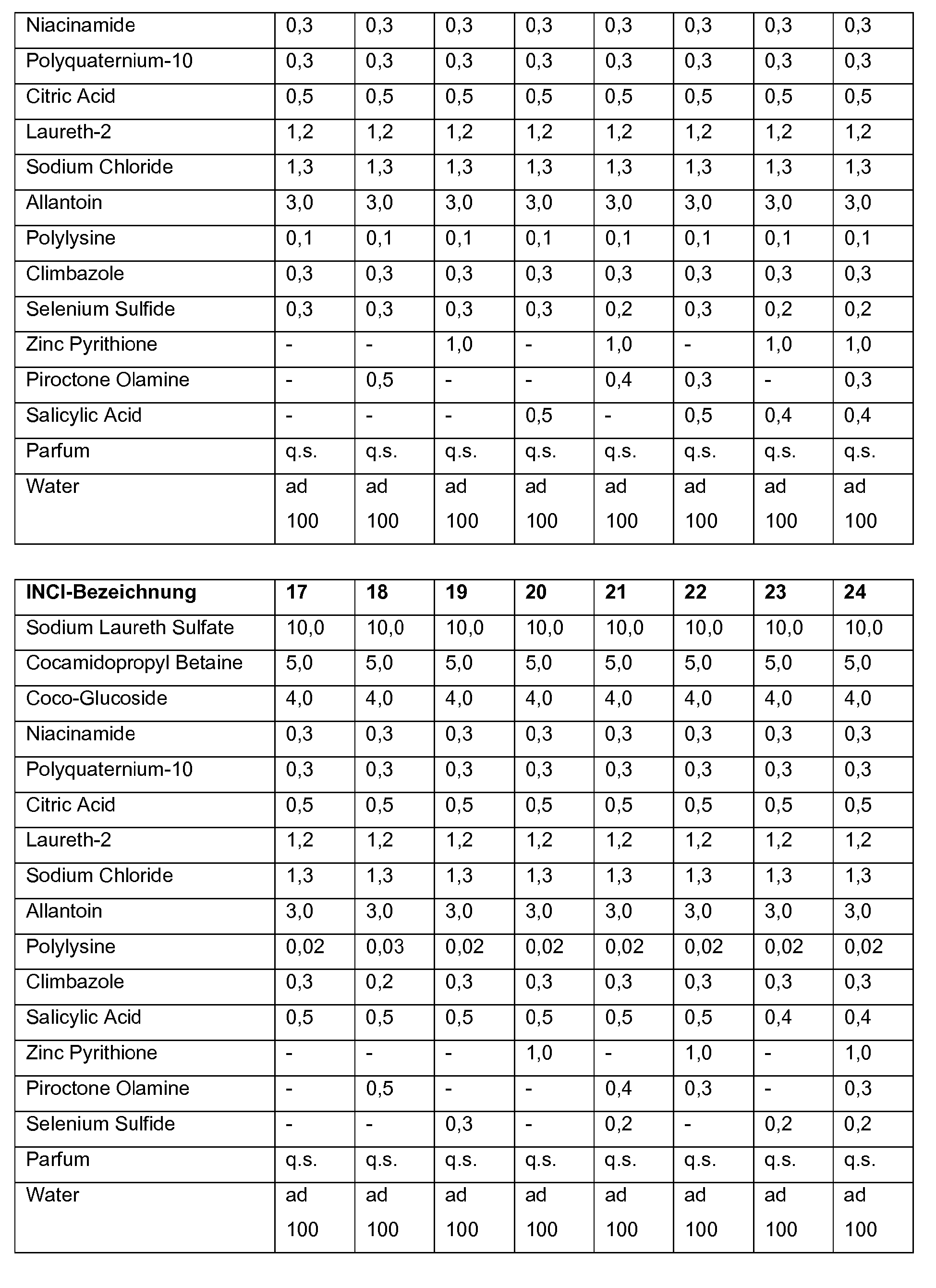

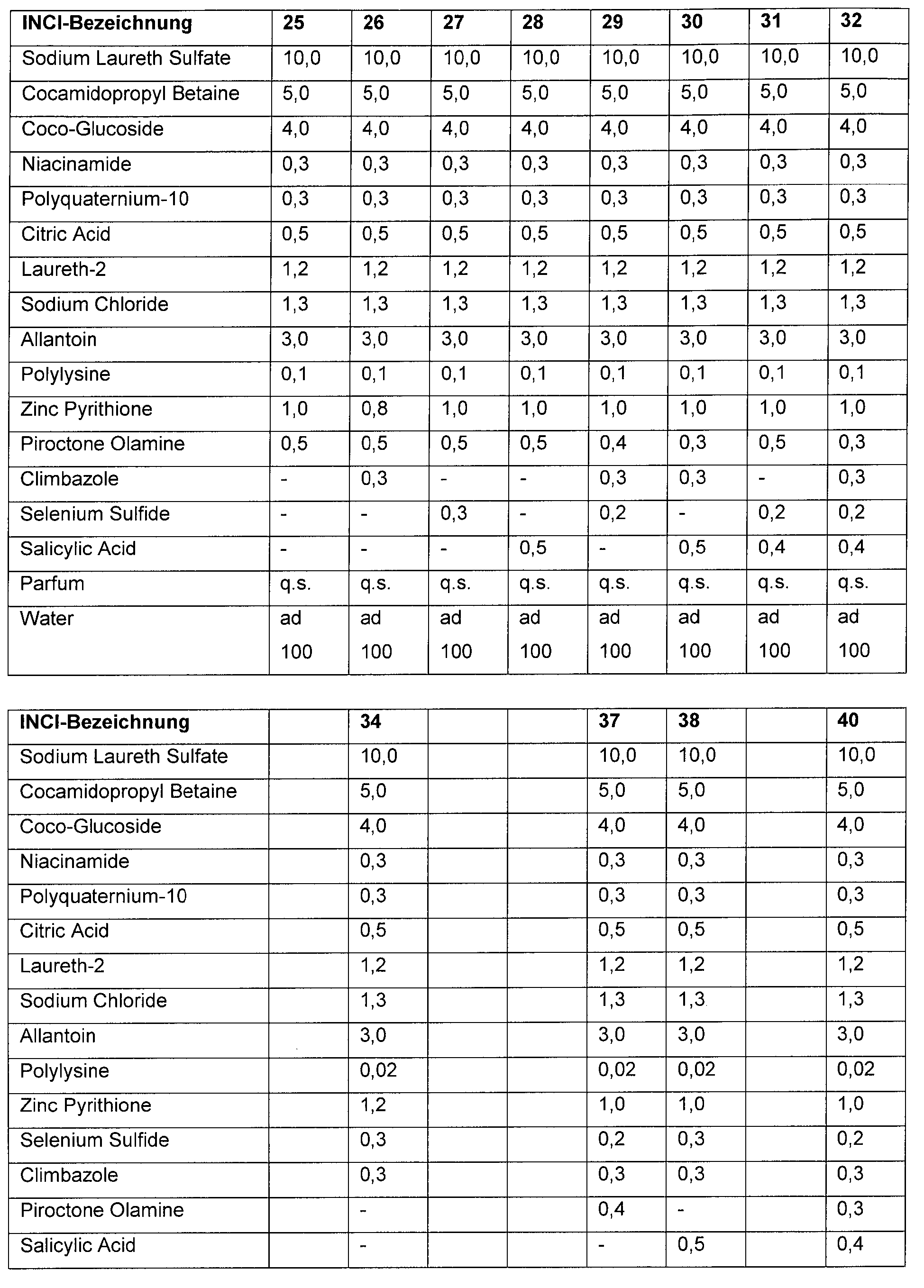

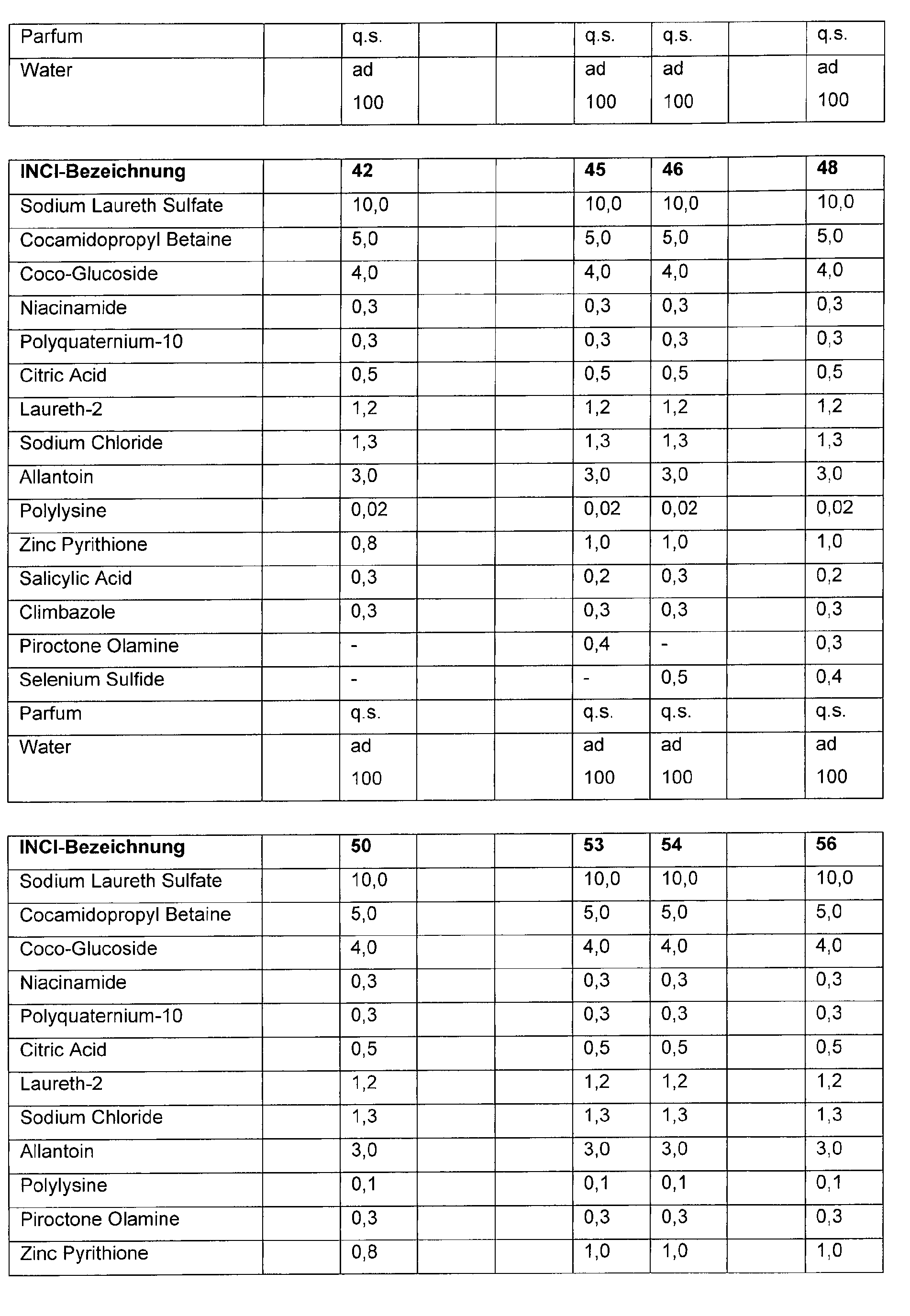

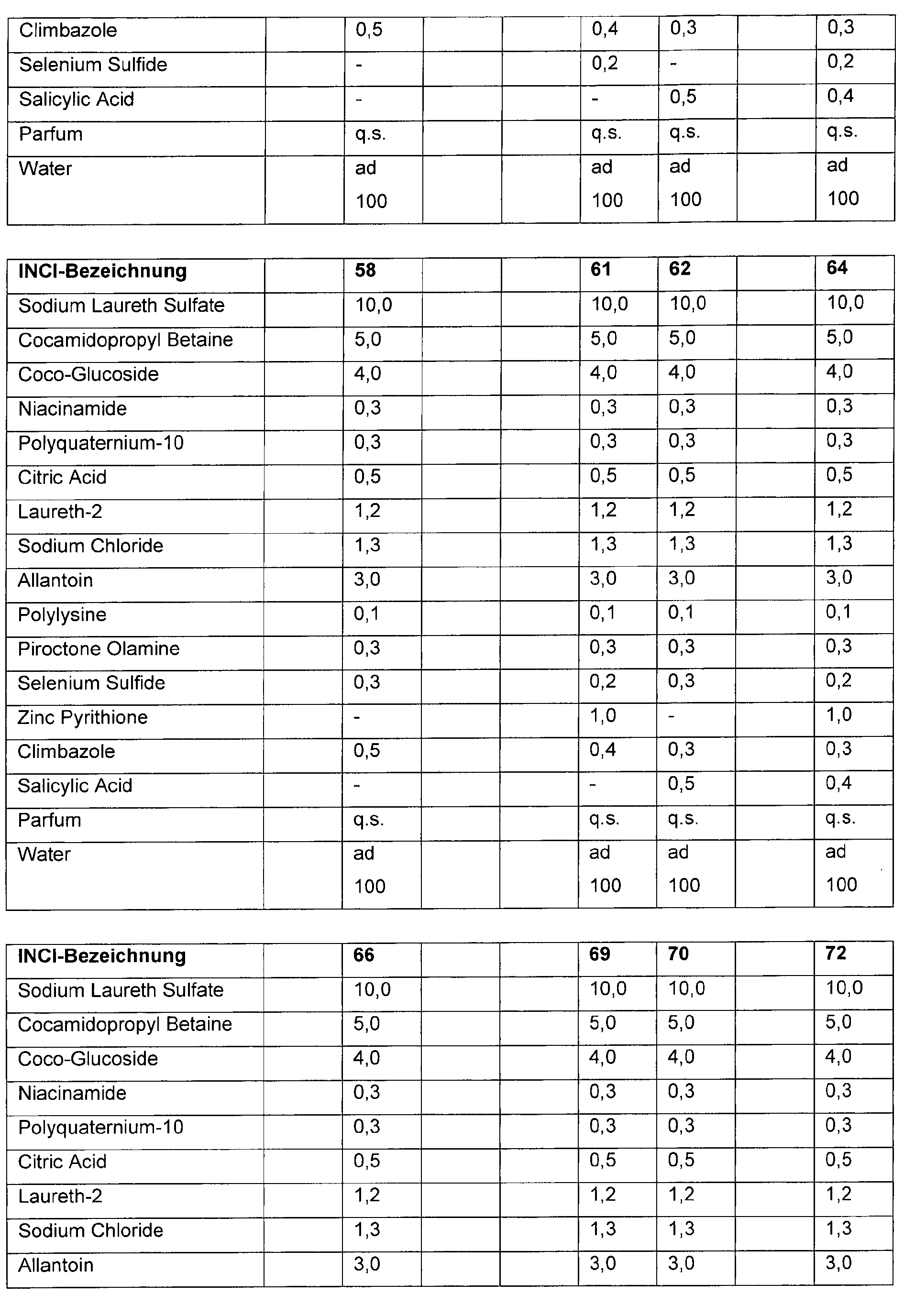

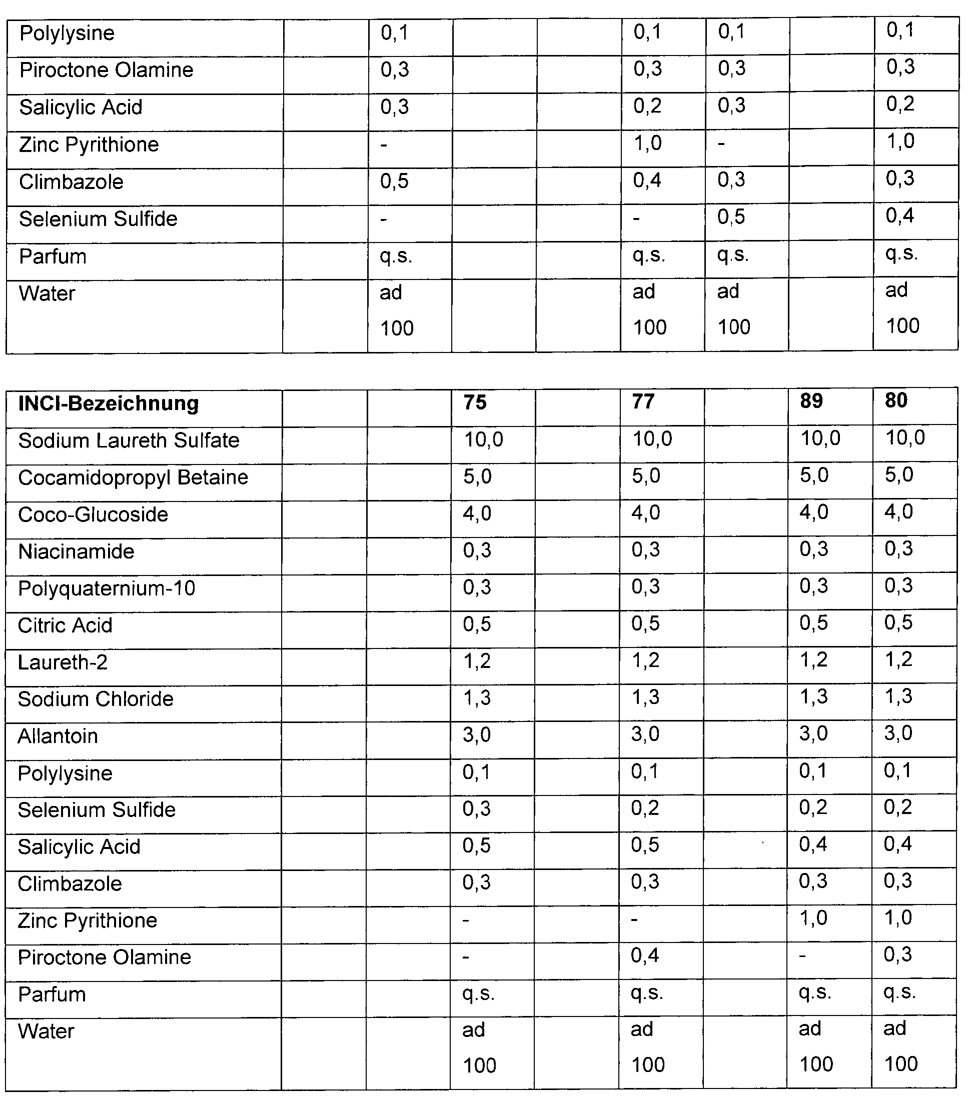

### 4. Haarshampoos:

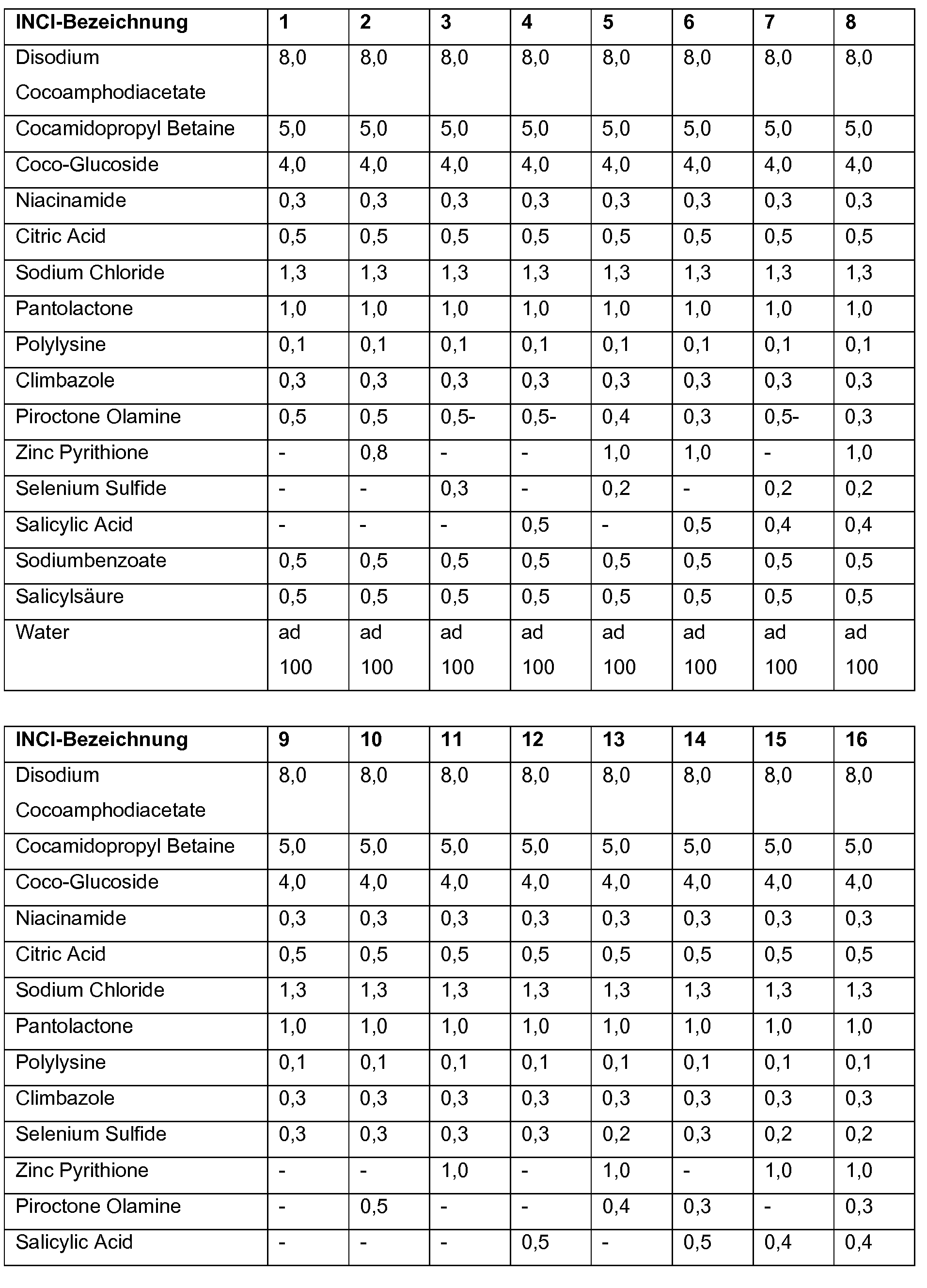

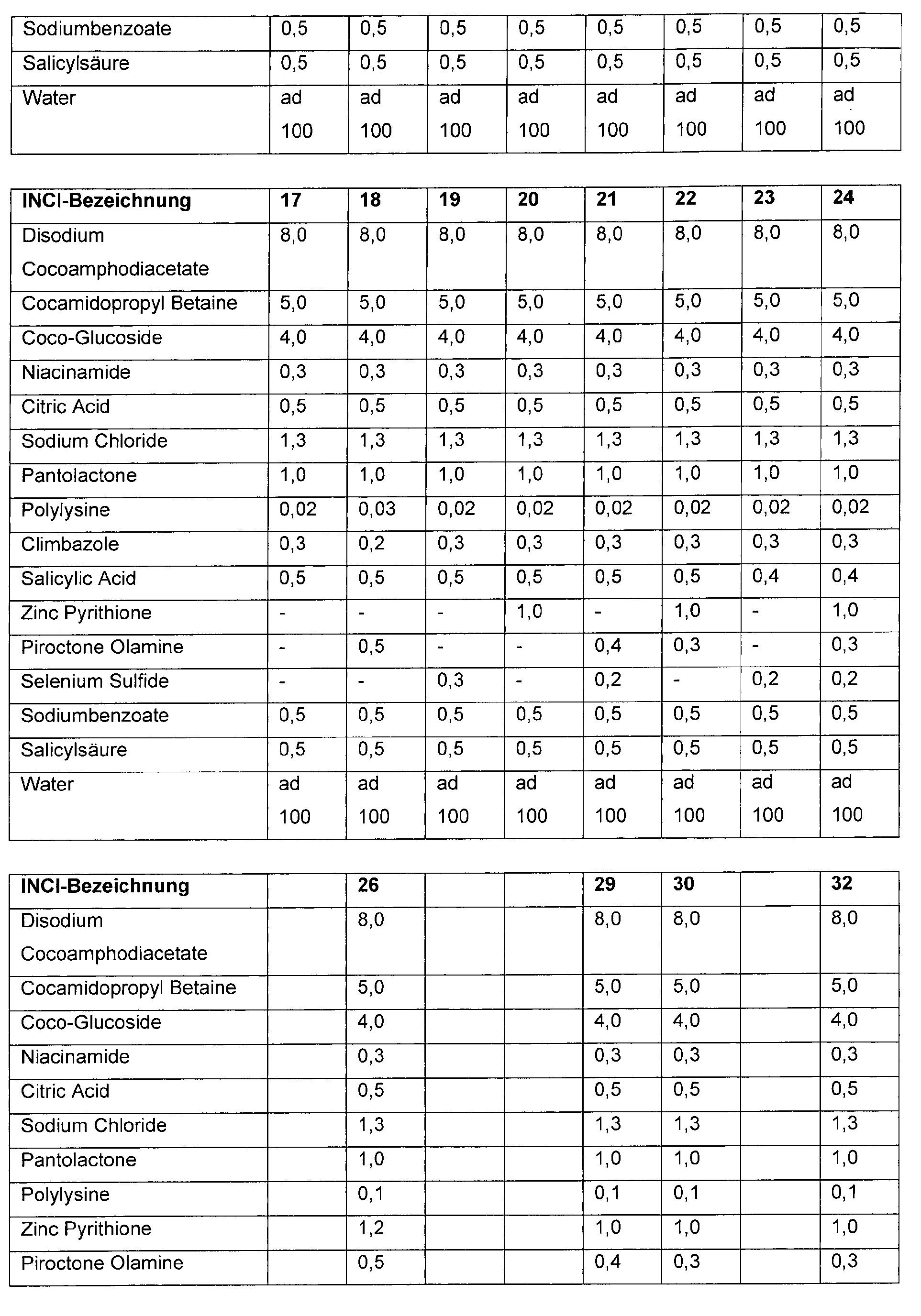

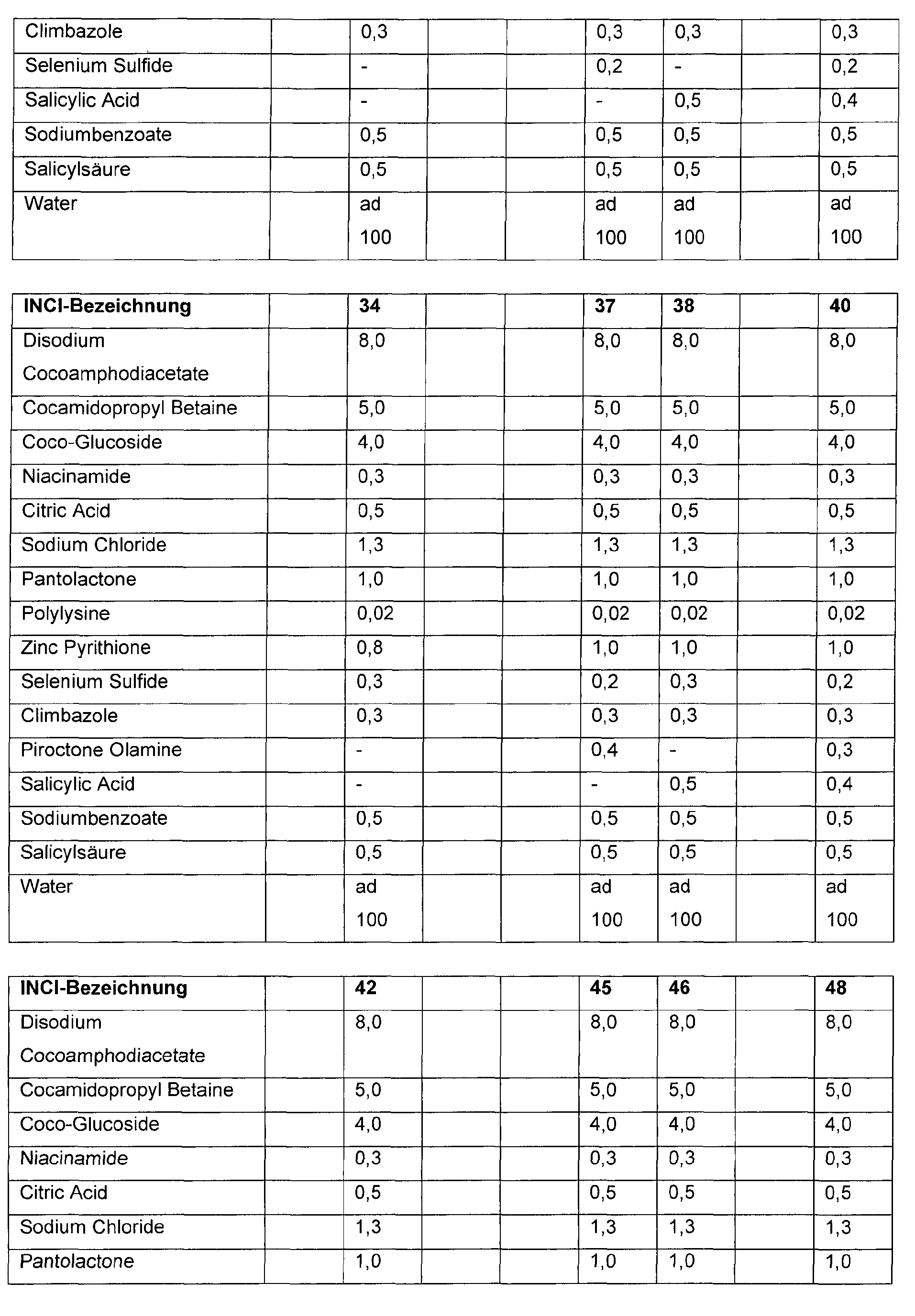

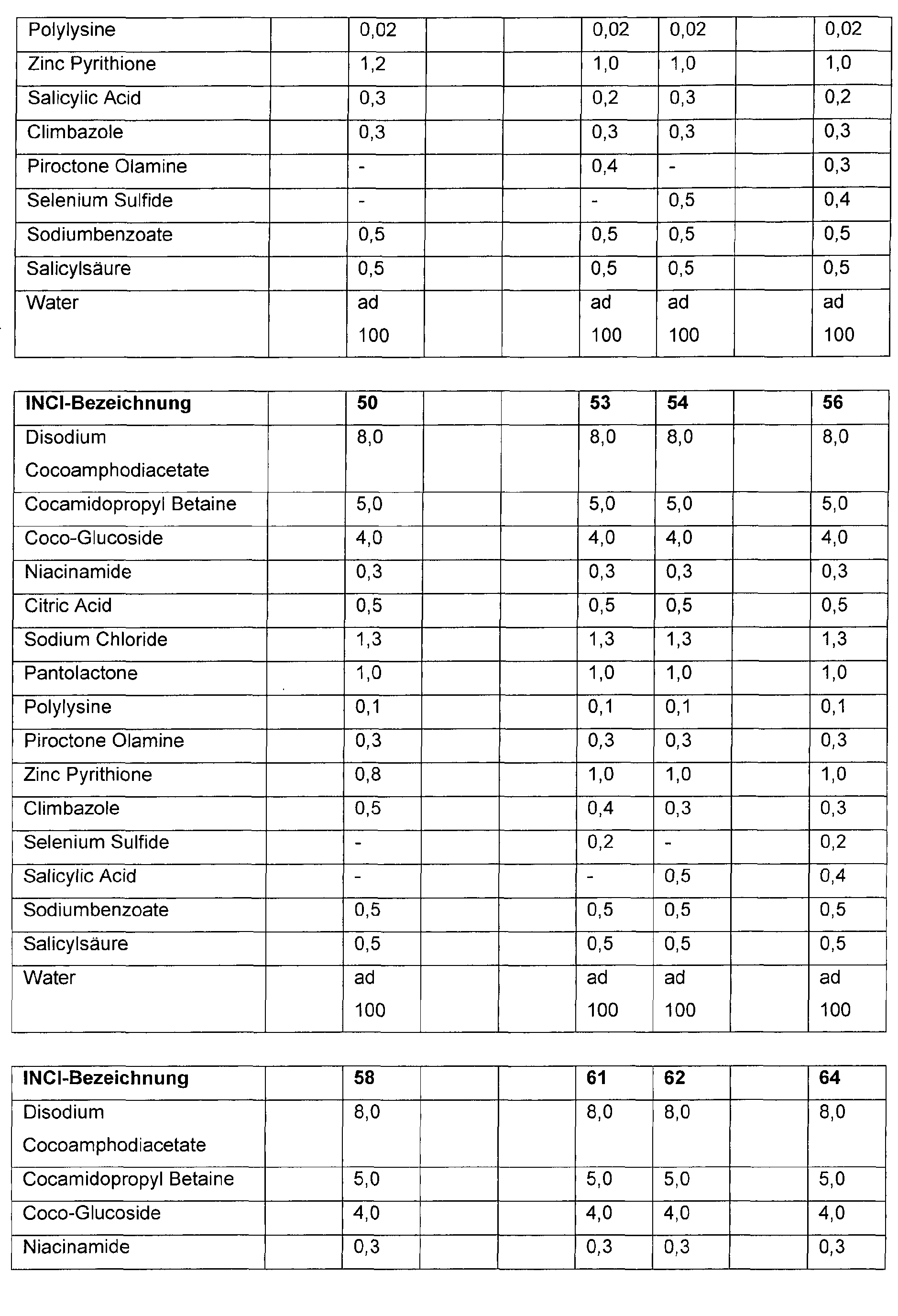

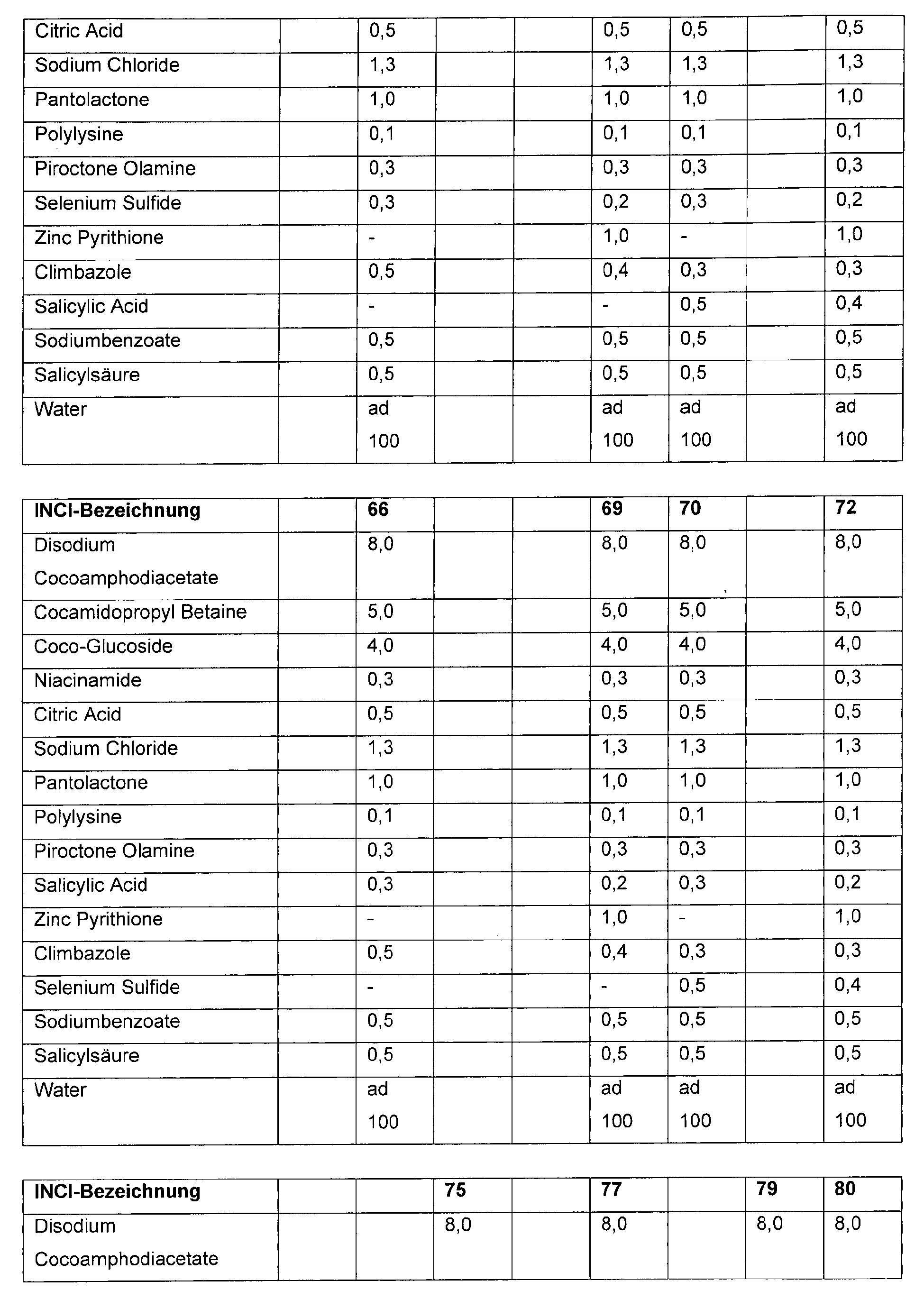

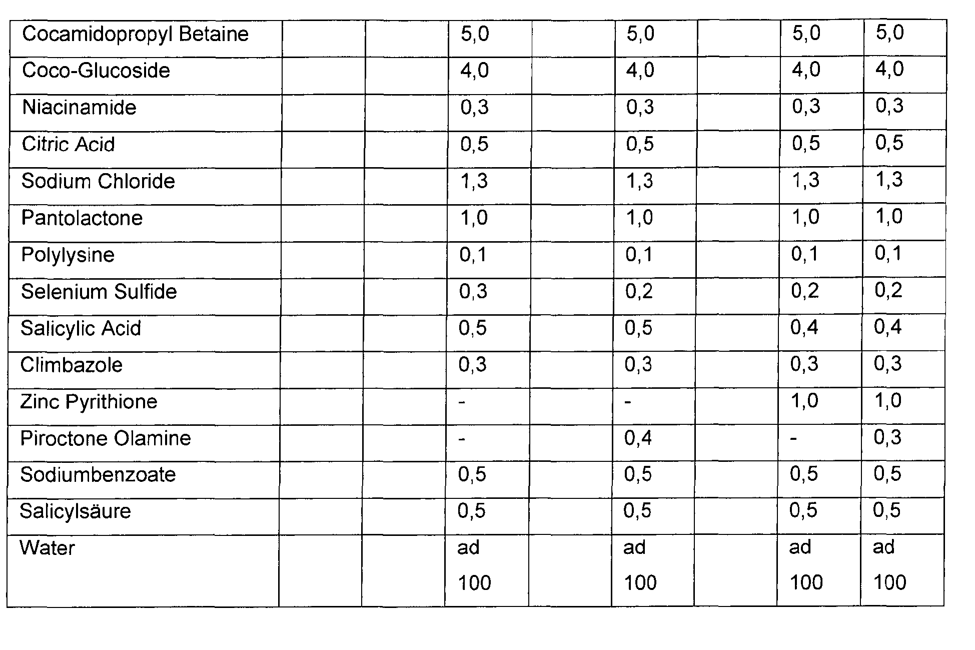

## Patentansprüche

1. Haarbehandlungsmittel, enthaltend - bezogen auf sein Gewicht -
a) 0,0001 bis 5 Gew.-% Epsilon-Poly-L-Lysin der allgemeinen Formel (I) 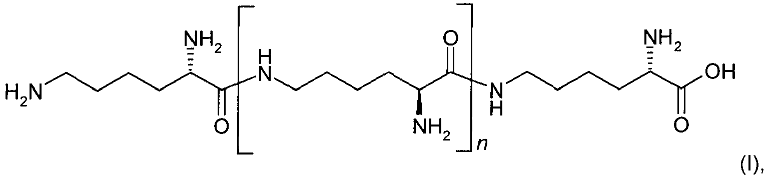 in der n für ganze Zahlen zwischen 5 und 100 steht,
b) 0,01 bis 10 Gew.-% eines Antischuppenwirkstoffes ausgewählt aus
b1) Climbazol
b2) Zinkpyrithion und/oder Zinkoxid
b3) Pirocton-Olamin
b4) Selensulfid(en)
b5) Salicylsäure
c) 0,01 bis 10 Gew.-% eines Antischuppenwirkstoffes, der von b) verschieden ist und ausgewählt ist aus
c1) Climbazol
c2) Zinkpyrithion und/oder Zinkoxid
c3) Pirocton-Olamin
c4) Selensulfid(en)
c5) Salicylsäure,
wobei das Mittel
- Epsilon-Poly-L-Lysin und Climbazol und Zinkpyrithion und/oder Zinkoxid
- Epsilon-Poly-L-Lysin und Climbazol und Pirocton-Olamin
- Epsilon-Poly-L-Lysin und Climbazol und Selensulfid(e)
enthält.

2. Haarbehandlungsmittel nach Anspruch 1, **dadurch gekennzeichnet, daß** es 0,0005 bis 4, vorzugsweise 0,001 bis 3, weiter bevorzugt 0,005 bis 2 und insbesondere 0,01 bis 1 Gew.-% Epsilon-Poly-L-Lysin der allgemeinen Formel (I) enthält.

3. Haarbehandlungsmittel nach einem der Ansprüche 1 oder 2, **dadurch gekennzeichnet, daß** es Epsilon-Poly-L-Lysin der allgemeinen Formel (I) enthält, in der n für ganze Zahlen von 10 bis 70, vorzugsweise von 15 bis 60, weiter bevorzugt von 20 bis 50 und insbesondere für die Zahlen 23, 24, 25, 26, 27, 28, 29, 30, 31, 32, 33, 34, 35 steht.

4. Haarbehandlungsmittel nach einem der Ansprüche 1 bis 3, **dadurch gekennzeichnet, daß** es 0,02 bis 7,5, vorzugsweise 0,03 bis 5, weiter bevorzugt 0,04 bis 2,5 und insbesondere 0,05 bis 0,5 Gew.-% Climbazol enthält.

5. Haarbehandlungsmittel nach einem der Ansprüche 1 bis 4, **dadurch gekennzeichnet, daß** es 0,02 bis 7,5, vorzugsweise 0,03 bis 5, weiter bevorzugt 0,04 bis 2,5 und insbesondere 0,05 bis 1 Gew.-% Zinkpyrithion und/oder Zinkoxid enthält.

6. Haarbehandlungsmittel nach einem der Ansprüche 1 bis 5, **dadurch gekennzeichnet, daß** es 0,01 bis 10 Gew.-%, vorzugsweise 0,02 bis 7,5, besonders bevorzugt 0,03 bis 5, weiter bevorzugt 0,04 bis 2,5 und insbesondere 0,05 bis 1 Gew.-% Pirocton-Olamin enthält.

7. Haarbehandlungsmittel nach einem der Ansprüche 1 bis 6, **dadurch gekennzeichnet, daß** es 0,01 bis 10 Gew.-%, vorzugsweise 0,02 bis 8, besonders bevorzugt 0,03 bis 6, weiter bevorzugt 0,04 bis 5 und insbesondere 0,05 bis 3 Gew.-% Selensulfid(e) enthält.

8. Haarbehandlungsmittel nach einem der Ansprüche 1 bis 7, **dadurch gekennzeichnet, daß** es 0,01 bis 10 Gew.-%, vorzugsweise 0,02 bis 8, besonders bevorzugt 0,03 bis 6, weiter bevorzugt 0,04 bis 5 und insbesondere 0,05 bis 2 Gew.-% Salicylsäure enthält.

## Claims

1. A hair treatment agent containing - based on its weight -
a) 0.0001 to 5 wt.% epsilon-poly-L-lysine of the general formula (I) 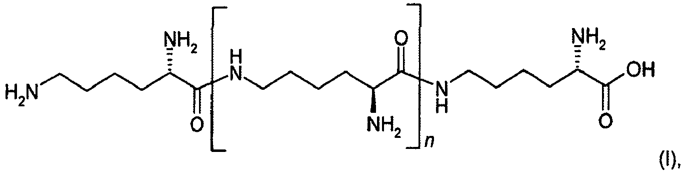 in which n represents integers of between 5 and 100,
b) 0.01 to 10 wt.% of an anti-dandruff active ingredient selected from
b1) climbazole
b2) zinc pyrithione and/or zinc oxide
b3) piroctone olamine
b4) selenium sulfide(s)
b5) salicylic acid
c) 0.01 to 10 wt.% of an anti-dandruff active ingredient which is different from b) and is selected from
c1) climbazole
c2) zinc pyrithione and/or zinc oxide
c3) piroctone olamine
c4) selenium sulfide(s)
c5) salicylic acid,
wherein the agent contains
- epsilon-poly-L-lysine and climbazole and zinc pyrithione and/or zinc oxide
- epsilon-poly-L-lysine and climbazole and piroctone olamine
- epsilon-poly-L-lysine and climbazole and selenium sulfide(s).

2. The hair treatment agent according to claim 1, **characterized in that** it contains 0.0005 to 4, preferably 0.001 to 3, more preferably 0.005 to 2 and in particular 0.01 to 1 wt.% epsilon-poly-L-lysine of the general formula (I).

3. The hair treatment agent according to claim 1 or 2, **characterized in that** it contains epsilon-poly-L-lysine of the general formula (I), in which n represents integers of from 10 to 70, preferably from 15 to 60, more preferably from 20 to 50 and in particular represents the numbers 23, 24, 25, 26, 27, 28, 29, 30, 31, 32, 33, 34, 35.

4. The hair treatment agent according to one of claims 1 to 3, **characterized in that** it contains 0.02 to 7.5, preferably 0.03 to 5, more preferably 0.04 to 2.5 and in particular 0.05 to 0.5 wt.% climbazole.

5. The hair treatment agent according to one of claims 1 to 4, **characterized in that** it contains 0.02 to 7.5, preferably 0.03 to 5, more preferably 0.04 to 2.5 and in particular 0.05 to 1 wt.% zinc pyrithione and/or zinc oxide.

6. The hair treatment agent according to one of claims 1 to 5, **characterized in that** it contains 0.01 to 10 wt.%, preferably 0.02 to 7.5, particularly preferably 0.03 to 5, more preferably 0.04 to 2.5 and in particular 0.05 to 1 wt.% piroctone olamine.

7. The hair treatment agent according to one of claims 1 to 6, **characterized in that** it contains 0.01 to 10 wt.%, preferably 0.02 to 8, particularly preferably 0.03 to 6, more preferably 0.04 to 5 and in particular 0.05 to 3 wt.% selenium sulfide(s).

8. The hair treatment agent according to one of claims 1 to 7, **characterized in that** it contains 0.01 to 10 wt.%, preferably 0.02 to 8, particularly preferably 0.03 to 6, more preferably 0.04 to 5 and in particular 0.05 to 2 wt.% salicylic acid.

## Revendications

1. Produit de traitement capillaire contenant - rapporté à son poids :
a) de 0,0001 à 5 % en poids de poly-ε-L-lysine, de formule générale (I) : 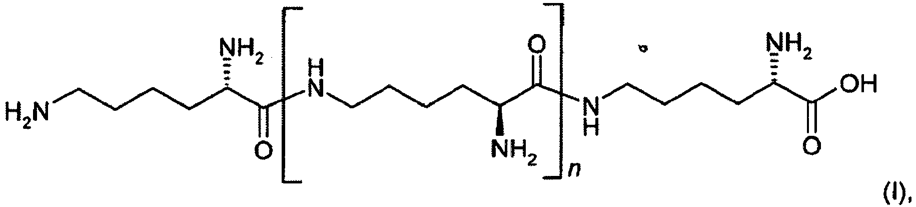 où n représente des nombres entiers entre 5 et 100,
b) de 0,01 à 10 % en poids d'un agent actif antipelliculaire choisi parmi :
b1) climbazole
b2) pyrithione de zinc et/ou oxyde de zinc
b3) piroctone olamine
b4) sulfure(s) de sélénium
b5) acide salicylique,
c) de 0,01 à 10 % en poids d'un agent actif antipelliculaire différent de b) et choisi parmi :
c1) climbazole
c2) pyrithione de zinc et/ou oxyde de zinc
c3) piroctone olamine
c4) sulfure(s) de sélénium
c5) acide salicylique,
le produit contenant :
- de la poly-ε-L-lysine, du climbazole et de la pyrithione de zinc et/ou de l'oxyde de zinc
- de la poly-ε-L-lysine, du climbazole et de la piroctone olamine
- de la poly-ε-L-lysine, du climbazole et du ou des sulfure(s) de sélénium.

2. Produit de traitement capillaire selon la revendication 1, **caractérisé en ce qu'**il contient de 0,0005 % à 4 %, préférentiellement de 0,001 % à 3 %, plus préférentiellement de 0,005 % à 2 % et en particulier de 0,01 % à 1 % en poids de ε-L-lysine de formule (I).

3. Produit de traitement capillaire selon l'une des revendications 1 ou 2, **caractérisé en ce qu'**il contient de la poly-ε-L-lysine de formule (I), où n représente des nombres entiers entre 10 et 70, de préférence entre 15 et 60, plus préférentiellement de 20 à 50, et en particulier les nombres 23, 24, 25, 26, 27, 28, 29, 30, 31, 32, 33, 34 et 35.

4. Produit de traitement capillaire selon l'une des revendications 1 à 3, **caractérisé en ce qu'**il contient de 0,02 % à 7,5 %, préférentiellement de 0,03 % à 5 %, plus préférentiellement de 0,04 % à 2,5 % et en particulier de 0,05 % à 0,5 % en poids de climbazole.

5. Produit de traitement capillaire selon l'une des revendications 1 à 4, **caractérisé en ce qu'**il contient de 0,02 % à 7,5 %, préférentiellement de 0,03 % à 5 %, plus préférentiellement de 0,04 % à 2,5 % et en particulier de 0,05 % à 1 % en poids de pyrithione de zinc et/ou oxyde de zinc.

6. Produit de traitement capillaire selon l'une des revendications 1 à 5, **caractérisé en ce qu'**il contient de 0,01 % à 10 %, préférentiellement de 0,02 % à 7,5 %, plus préférentiellement de 0,03 % à 5 %, particulièrement préférentiellement de 0,04 % à 2,5 % et en particulier de 0,05 % à 1 % en poids de piroctone olamine.

7. Produit de traitement capillaire selon l'une des revendications 1 à 6, **caractérisé en ce qu'**il contient de 0,01 % à 10 %, préférentiellement de 0,02 % à 8 %, plus préférentiellement de 0,03 % à 6 %, particulièrement préférentiellement de 0,04 % à 5 % et en particulier de 0,05 % à 3 % en poids de sulfure(s) de sélénium.

8. Produit de traitement capillaire selon l'une des revendications 1 à 7, **caractérisé en ce qu'**il contient de 0,01 % à 10 %, préférentiellement de 0,02 % à 8 %, plus préférentiellement de 0,03 % à 6 %, particulièrement préférentiellement de 0,04 % à 5 % et en particulier de 0,05 % à 2 % en poids d'aide salicylique.
